# NEW EUROPEAN PATENT SPECIFICATION

(11) **EP 0 093 619 B2**
(45) Date of publication and mention of the opposition decision: **18.09.1996**
(45) Mention of the grant of the patent: 13.09.1989
(21) Application number: 83302501.8
(22) Date of filing: 04.05.1983
(51) Int. Cl.: C12N 15/00, C12N 9/48, C12N 9/64, A61K 38/46

(54) **Human tissue plasminogen activator, pharmaceutical compositions containing it, processes for making it, and DNA and transformed cell intermediates therefor**
Plasminogenaktivator für menschliches Gewebe, diesen Aktivator enthaltende pharmazeutische Zusammensetzungen, Verfahren zur Herstellung des Aktivators sowie DNA und transformierte Zellzwischenprodukte hierfür
Plasminogène-activateur de tissu humain, compositions pharmaceutiques le contenant, procédés pour sa préparation ainsi que ADN et intermédiaires cellulaires transformés pour la mise en oeuvre de ces procédés

(30) Priority: 05.05.1982 US 374860; 14.07.1982 US 398003; 07.04.1983 US 483052
(43) Date of publication of application: 09.11.1983
(73) Proprietor: GENENTECH, INC., South San FranciscoCalifornia 94080 (US)
(72) Inventor: Goeddel, David VanNorman, HillsboroughCalifornia 94010 (US); Kohr, William Jack, San MateoCalifornia 94403 (US); Pennica, Diane, Foster CityCalifornia 94404 (US); Vehar, Gordon Allen, San CarlosCalifornia 94070 (US)
(74) Representative: Armitage, Ian Michael

(56) References cited:
- EP-A- 0 023 860
- EP-A- 0 037 687
- EP-A- 0 041 766
- US-A- 4 245 051
- THE JOURNAL OF BIOLOGICAL CHEMISTRY, vol. 257, no. 6, March 25, 1982, USA. M. HOYLAERTS et al.: "Kinetics of the Activation of Plasminogen by Human Tissue Plasminogen Activator", pp. 2912-2918
- THE LANCET, November 7, 1981, London, Boston. W. WEIMAR et al.: "Specific Lysis of an Iliofemoral Thrombus by Administration of Extriusic (Tissue-Type) Plasminogen Activator", pp. 1018-1020
- NATURE, vol. 272, no. 5648, March 2, 1978, USA. B. WIMAN, D. COLLEN: "Molecular mechanism of physiological fibrinolysis", pp. 549, 550

## Description

The present invention relates to human plasminogen activator, corresponding to that found in human serum and/ or tissues, and to novel forms and compositions thereof and particularly to the means and methods for its production to homogeneity in therapeutically significant quantities.

The present invention arises in part from the discovery of the DNA sequence and deduced amino acid sequence of human plasminogen activator. This discovery enables the production of human plasminogen activator via the application of recombinant DNA technology, in turn, enabling the production of sufficient quality and quantity of material to initiate and conduct animal and clinical testing as prerequisites to market approval, unimpeded by the restrictions necessarily inherent in the isolation methods hitherto employed involving production and extraction from existing cell culture. This invention is directed to these associated embodiments in all respects.

The publications and other materials hereof used to illuminate the background of the invention, and in particular cases, to provide additional details concerning its practice are incorporated herein by reference, and for convenience, are numerically referenced in the following text and respectively grouped in the appended bibliography.

### Background of the invention

### A. Human tissue plasminogen activator

The fibrinolytic system is in a dynamic equilibrium with the coagulation system, maintaining an intact, patent vascular bed. The coagulation system deposits fibrin as a matrix serving to restore a hemostatic condition. The fibrinolytic system removes the fibrin network after the hemostatic condition is achieved. The fibrinolytic process is brought about by the proteolytic enzyme plasmin that is generated from a plasma protein precursor plasminogen. Plasminogen is converted to plasmin through activation by an activator.

Currently, two activators are commercially available, streptokinase and urokinase. Both are indicated for the treatment of acute vascular diseases such as myocardial infarct, stroke, pulmonary embolism, deep vein thrombosis, peripheral arterial occlusion and other venous thromboses. Collectively, these diseases account for major health hazards and risks.

The underlying etiological basis for these diseases points to either a partial, or in severe cases, total occlusion of a blood vessel by a blood clot-thrombus or thromboembolus. Traditional anticoagulant therapy, as with heparin and coumarin, does nothing to directly enhance dissolution of thrombi or thromboemboli. The thrombolytic agents referred to earlier, streptokinase and urokinase, have enjoyed practical and effective use. However, each has severe limitations. Neither has a high affinity for fibrin; consequently, both activate circulating and fibrin-bound plasminogen relatively indiscriminately. The plasmin formed in circulating blood is neutralized rather quickly and lost for useful thrombolysis. Residual plasmin will degrade several clotting factor proteins, for example, fibrinogen, Factor V and Factor VIII, causing a hemorrhagic potential. In addition, steptokinase is strongly antigenic and patients with high antibody titers respond inefficiently to treatment and cannot remain on continuous treatment. Urokinase therapy is expensive, owing to its involved isolation from human urine or tissue culture, and it, therefore, is not generally accepted in clinical practice. Urokinase has been the subject of numerous investigations-See, for example, references 1-6.

So-called plasminogen activators have been isolated from various human tissue, e.g., uterine tissue, blood, serum-see generally references 7-11 and from cell culture (reference 94). Compositions thereof have also been described-see references 12, 13. See also references 14-18. The plasminogen activators derived from these sources have been classified into two major groups: urokinase-type plasminogen activators (u-PA) and tissue-type plasminogen activators (t-PA) based on differences in their immunological properties. (The abbreviations t-PA and u-PA are those proposed at the XXVIII Metering of the International Committee on Thrombosis and Hemostasis, Bergamo, Italy, 27 July 1982).

Recently, a human melanoma line has been identified which secretes t-PA. Characterization of this melanoma plasminogen activator has shown it to be indistinguishable both immunologically and in amino acid composition from the plasminogen activator isolated from normal human tissue (Reference 19, 88).

The product was isolated in relatively pure form, characterized and found to be a highly active fibrinolytic agent (20).

Several studies (eg. References 95 to 98) which used t-PA purified from the melanoma cell line have demonstrated its higher affinity for fibrin, compared with urokinase type plasminogen activators. More by intensive investigation of human t-PA as a potential thrombolytic agent has, however, been hampered by its extremely low concentration in blood, tissue extracts, vessel perfusates and cell cultures.

It was perceived that the application of recombinant DNA and associated technologies would be a most effective way of providing the requisite large quantities of high quality human tissue-type plasminogen activator (earlier referred to as human plasminogen activator), essentially free of other human protein. Such materials would probably exhibit bioactivity admitting of their use clinically in the treatment of various cardiovascular conditions or diseases.

### B. Recombinant DNA technology

Recombinant DNA technology has reached the age of some sophistication. Molecular biologists are able to recombine various DNA sequences with some facility, creating new DNA entities capable of producing copious amounts of exogenous protein product in transformed microbes and cell cultures. The general means and methods are in hand for in in vitro ligation of various blunt ended or "sticky" ended fragments of DNA, producing potent expression vehicles useful in transforming particular organisms, thus directing their efficient synthesis of desired exogenous product. However, on an individual product basis, the pathway remains somewhat tortuous and the science has not advanced to a stage where regular predictions of success can be made. Indeed, those who portend successful results without the underlying experimental basis, do so with considerable risk of inoperability

DNA recombination of the essential elements, i.e., an origin of replication, one or more phenotypic selection characteristics, an expression promoter, heterologous gene insert and remaining vector, generally is performed outside the host cell. The resulting recombinant replicable expression vehicle, or plasmid, is introduced into cells by transformation and large quantities of the recombinant vehicle obtained by growing the transformant. Where the gene is properly inserted with reference to portions which govern the transcription and translation of the encoded DNA message, the resulting expression vehicle is useful to actually produce the polypeptide sequence for which the inserted gene codes, a process referred to as expression. The resulting product may be obtained by lysing, if necessary, the host cell, in microbial systems, and recovering the product by appropriate purification from other proteins.

In practice, the use of recombinant DNA technology can express entirely heterologous polypeptides-so-called direct expression-or alternatively may express a heterologous polypeptides fused to a portion of the amino acid sequence of a homogolous polypeptide. In the latter cases, the intended bioactive product is sometimes rendered bioinactive within the fused, homologous/heterologous polypeptide until it is cleaved in an extracellular environment. See references (21 ) and (22).

Similarly, the art of cell or tissue cultures for studying genetics and cell physiology is well established. Means and methods are in hand for maintaining permanent cell lines, prepared by successive serial transfers from isolate normal cells. For use in research, such cell lines are maintained on a solid support in liquid medium, or by growth in suspension containing support nutriments. Scale-up for large preparations seems to pose only mechanical problems. For further background, attention is directed to references (23) and (24).

Likewise, protein biochemistry is a useful, indeed necessary, adjunct in biotechnology. Cells producing the desired protein also produce hundreds of other proteins, endogenous products of the cell's metabolism. These contaminating proteins, as well as other compounds, if not removed from the desired protein, could prove toxic if administered to an animal or human in the course of therapeutic treatment with desired protein. Hence, the techniques of protein biochemistry come to bear, allowing the design of separation procedures suitable for the particular system under consideration and providing a homogeneous product safe for intended use. Protein biochemistry also proves the identity of the desired product, characterizing it and ensuring that the cells have produced it faithfully with no alterations or mutations. This branch of science is also involved in the design of bioassays, stability studies and other procedures necessary to apply before successful clinical studies and marketing can take place.

### Summary of the invention

The present invention is based upon the discovery that recombinant DNA technology can be used successfully to produce human tissue plasminogen activator (t-PA), preferably in direct form, and in amounts sufficient to initiate and conduct animal and clinical testing as prerequisites to market approval. The product human t-PA is suitable for use, in all of its forms, in the prophylactic or therapeutic treatment of human beings for various cardiovascular conditions or diseases. Accordingly, the present invention, in one important aspect, is directed to methods of treating vascular disorders in human subjects using t-PA and to suitable pharmaceutical compositions thereof.

The present invention discloses for the first time a polypeptide of specified amino-acid sequence which has human tissue plasminogen activator function, and which can be expressed in a recombinant host cell from DNA encoding the polypeptide. Such a coding sequence is also disclosed for the first time. Variations or modifications in the polypeptide, as natural alleles or other derivatives, are possible while retaining the plasminogen activator function. Thus, the present invention, in enabling the production of such polypeptides by genetically engineered microorganisms or cell culture systems provides an opportunity to produce human tissue plasminogen activator in a much more efficient manner than has been possible, enabling hitherto elusive commercial exploitation. In addition, depending upon the host cell, the human tissue plasminogen activator hereof may contain associated glycosylation to a greater or lesser extent compared with native material. In any event, the t-PA will be free of the contaminants normally associated with it in its non-recombinant cellular environment.

The present invention is also directed to recombinant DNA expression vectors hardening DNA sequences encoding such polypeptides in expressible form, and to recombinant microorganisms or cell cultures capable of expressing such polypeptides. In still further aspects the invention is directed to processes for preparing such recombinant microorganisms and cell cultures, and to processes for preparing such polypeptides by expression in a recombinant host cell of DNA encoding the polypeptide.

In the accompanying drawings:
Figure 1 shows a 10% sodium dodecylsulfate polyacrylamide gel electrophoresis (SDS PAGE) of ³⁵S-methionine labelled proteins precipitable with anti t-PA IgG secreted from melanoma cells with and without protease inhibitor.
Figure 2 shows electrophoresis of the immuno-precipitated translation products of mRNA fractions derived from melanoma cells.
Figure 3 shows the hybridization pattern of 96 bacterial colonies transformed with cDNA using the pool of ^{32p} labelled 14-mer as probe prepared based on a 5 amino acid sequence of human t-PA.
Figure 4 is a restriction endonuclease map of the full length human t-PA cDNA.
Figures 5a, 5b, and 5c show the nucleotide sequence and deduced amino acid sequence of the full length human t-PA cDNA.
Figure 6 is a schematic of the construction of the expression plasmid _{P}ARIPA'.
Figure 7 shows the results of a fibrin plate assay for fibrinolytic activity of E. coli cells transformed with pORIPA°.
Figure 8 is an HPLC trace of peptides from human t-PA trypsin digest
Figure 9 shows the construction of a plasmid coding for the direct expression of mature buman t-PA in E. coli.
Figure 10 shows the results of a fibrin plate assay for fibrinolytic activity of the human t-PA produced by E. coli transformed with pt-PAtrpl2.

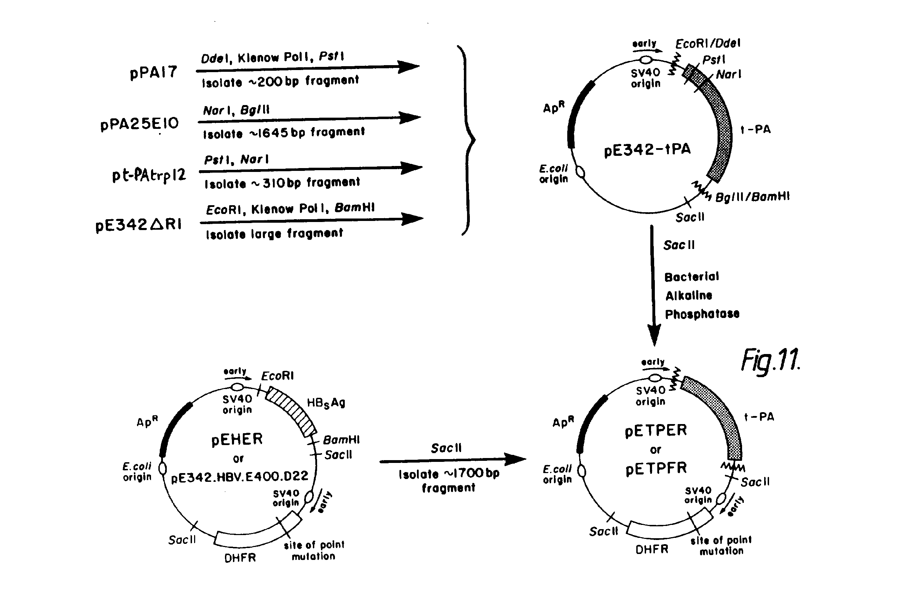

Figure 11 shows the construction of DHFR (mutant or wild typeyt-PA encoding plasmids suitable for transforming into mammalian tissue culture cells.
Figure 12 is a schematic diagram of human tissue plasminogen activator as prepared by the method exemplified in E.1 herein.

### A. Definitions

As used herein, "human tissue plasminogen activator" or "human t-PA" or "t-PA" denotes human extrinsic (tissue-type) plasminogen activator, produced by microbial or cell culture systems, in bioactive forms comprising a protease portion and corresponding to those tissue plasminogen activators otherwise native to human tissue. The human tissue plasminogen activator protein produced herein has been defined by means of determined DNA gene and deductive amino acid sequencing. It will be understood that natural allelic variations exist and occur from individual to individual. These variations may be demonstrated by (an) amino acid difference(s) in the overall sequence or by deletions, substitutions, insertions, inversions or additions of (an) amino acid(s) in said sequence. In addition, the location of and degree of glycosylation will depend on the nature of the host cellular environment.

The potential exists, in the use of recombinant DNA technology, for the preparation of various human tissue plasminogen activator derivatives, variously modified by resultant single or multiple amino acid substitutions, deletions, additions or replacements, for example, by means of site directed mutagenesis of the underlying DNA. Included would be the preparation of derivatives retaining the essential kringle region and serine protease region characteristic generally of the human tissue plasminogen activator described specifically herein, but otherwise modified as described above. All such allelic variations and modifications resulting in derivatives of human tissue plasminogen activator are included within the scope of this invention, as well as other related human extrinsic (tissue-type) plasminogen activators, similar physically and biologically, so long as the essential, characteristic human tissue plasminogen activator activity remains unaffected in kind.

Human tissue plasminogen activator is prepared 1 ) having methionine as its first amino acid (present by virtue of the ATG start signal codon insertion in front of the structural gene) or 2) where the methionine is intra- or extracellularly cleaved, having its normally first amino acid, or 3) together with either its signal polypeptide or a conjugated protein other than the conventional signal polypeptide, the signal polypeptide or conjugate being specifically cleavable in an intra- or extracellular environment (See reference 21), or 4) by direct expression in mature form without the necessity of cleaving away any extraneous, superflous polypeptide. The latter is particularly important where a given host may not, or not efficiently, remove a signal peptide where the expression vehicle is designed to express the tissue plasminogen activator together with its signal peptide. In any event, the thus produced human t-PA, in its various forms, is recovered and purified to a level fitting it for use in the treatment of various vascular conditions or diseases.

Furthermore, t-PA has forms which include both the single chain (1-chain) protein and the 2-chain protein. The latter is proteolytically derived from the 1-chain compound. It is theorized that the 2-chain protein is associated with produced fibrin and that proteolytic conversion from 1- or 2- chain material occurs at the locus of the conversion of plasminogen to plasmin. The present invention provides for the administration of the 1-chain protein for in vivo conversion as just described or for the administration of 2-chain protein, which has also been shown to be active. The 2-chain protein can be prepared by in vitro proteolytic conversion after the 1-chain material is produced. A so-called "kringle" area is positioned upstream from the serine protease portion and is believed to play an impormant function in binding the tissue plasminogen activator hereof to a fibrin matrix; hence, the observed specific activity of the present tissue plasminogen activator toward tangible, extent thrombi. The tissue plasminogen activator hereof is produced containing the enzymatically active portion corresponding to native material and the term human tissue plasminogen activator defines products comprising such portion alone or together with additional amino acid sequences up to the full length molecule.

To summarize in the present invention, human t-PA thus has a functional definition; it is capable of catalyzing the conversion of plasminogen to plasmin, binds to fibrin, and is classified as a t-PA based on immunological properties as set forth hereinabove.

"Essentially pure form" when used to describe the state of human t-PA produced by the invention means free of protein or other materials normally associated with human t-PA when produced by non-recombinant cells, i.e. in its "native" environment.

"DHFR protein" refers to a protein which is capable of the activity associated with dihydrofolate reductase (DHFR) and which, therefore, is required to be produced by cells which are capable of sunrival on medium deficient in hypoxanthine, glycine, and thymidine (-HGT medium). In general, cells lacking DHFR protein are incapable of growing on this medium, cells which contain DHFR protein are successful in doing so.

"Cells sensitive to MTX" refers to cells which are incapable of growing on media which contain the DHFR inhibitor methotrexate (MTX). Thus, "cells sensitive to MTX" are cells which, unless genetically altered or otherwise supplemented, will fail to grow under ambient and medium conditions suitable for the cell type when the MTX concentration is 0.2 pg/ml or more. Some cells, such as bacteria, fail to exhibit MTX sensitivity due to their failure to permit MTX inside their cell boundaries, even though they contain DHFR which would otherwise be sensitive tothis drug. In general, cells which contain, as their DHFR protein, wild type DHFR will be sensitive to methotrexate if they are permeable or capable of uptake with respect to MTX.

"Wild type DHFR" refers to dihydrofolate reductase as is ordinarily found in the particular organism in question. Wild type DHFR is generally sensitive in vitro to low concentrations of methotrexate.

"DHFR protein with low binding affinity for MTX" has a functional definition. This is a DHFR protein which, when generated within cells, will permit the growth of MTX sensitive cells in a medium containing 0.2 pg/ml or more of MTX. It is recognized that such a functional definition depends on the facility with which the organism produces the "DHFR protein with low binding affinity for MTX" as well as upon the protein itself. However, as used in the context of this invention, such a balance between these two mechanisms should not be troublesome. The invention operates with respect to conferring the capability of surviving these levels of MTX, and it is not consequential whether the ability to do so is impacted by increased expression in addition to the innate nature of the DHFR produced. A convenient DHFR protein which fits this definition is disclosed in U.S. Appl Serial No. 459, 151, filed January 19, 1983, incorporated herein by reference.

"Expression vector" includes vectors which are capable of expressing DNA sequences contained therein, where such sequences are operably linked to other sequences capable of effecting their expression. It is implied, although not always explicitly stated, that these expression vectors must be replicable in the host organisms either as episomes or as an integral part of the chromosomal DNA. Clearly a lack of replicability would render them effectively inoperable. In sum, "expression vector" is given a functional definition, and any DNA sequence which is capable of effecting expression of a specified DNA code disposed therein is included in this term as it is applied to the specified sequence. In general, expression vectors of utility in recombinant DNA techniques are often in the form of "plasmids" which refer to circular double stranded DNA loops which, in their vector form are not bound to the chromosome. In the present specification, "plasmid" and "vector" are used interchangeably as the plasmid is the most commonly used form of vector. However the invention is intended to include such other forms of expression vectors which serve equivalent functions and which become known in the art subsequently hereto.

"Recombinant host cells" refers to cells which have been transformed with vectors constructed using recombinant DNA techniques. As defined herein, t-PA is produced in the amounts achieved by virtue of this transformation, rather than in such lesser amounts, or, more commonly, in such less than detectable amounts, as might be produced by the untransformed host. t-PA produced by such cells can be referred to as "recombinant t-PA".

### B. Host cell cultures and vectors

The vectors and methods disclosed herein are suitable for use in host cells over a wide range of prokaryotic and eukaryotic organisms.

In general, of course, prokaryotes are preferred for cloning of DNA sequences in constructing the vectors useful in the invention. For example, E. coli K12 strain 294 (ATCC No. 31446) is particularly useful. Other microbial strains which may be used include E. coli strains such as E. coli B, and E. coli X1776 (ATTC No. 31537). These examples are, of course, intended to be illustrative rather than limiting.

Prokaryotes may also be used for expression. The aforementioned strains, as well as E. coli W3110(F-, λ⁻, pro-totrophic, ATCC No. 27325), bacilli such as Bacillus subtilus, and other enterobacteriaceae such as Salmonella typh-imurium or Serratia marcescens, and various pseudomonas species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR 322, a plasmid derived from an E. coli species (Bolivar, et al., Gene, 2:95 (1977)). pBR 322 contains genes for ampicillin and tetracycline resistance and thus provides easy means for identifying transformed cells. The pBR 322 plasmid, or other microbial plasmid must also contain, or be modified to contain, promoters which can be used by the microbial organism for expression of its own proteins. Those promoters most commonly used in recombinant DNA construction include the β-lactamase (penicillinase) and lactose promoter systems (Chang et al., Nature, 275: 617 (1978); Itakura, et al, Science, 198: 1056 (1977); (Goeddel, et al Nature 281: 544 (1979) and a tryptophan (trp) promoter system (Goeddel, et al, Nucleic Acids Res., 8: 4057 (1980); EPO Appl Publ No. 0036776). While these are the most commonly used, other microbial promoters have been discovered and utilized, and details concerning their nucleotide sequences have been published, enabling a skilled worker to ligate them functionally with plasmid vectors (Siebenlist, et al, Cell 20: 269 (1980)).

In addition to prokaryotes, eukaryotic microbes, such as yeast cultures may also be used. Saccharomyces cere-visiae, or common baker's yeast is the most commonly used among eukaryotic microorganisms, although a number of other strains are commonly available. For expression in Saccharomyces, the plasmid YRp7, for example, (Stinchcomb, et al, Nature, 282:39 (1979); Kingsman et al, Gene, 7: 141(1979); Tschemper, et al, Gene, 10: 157 (1980)) is commonly used. This plasmid already contains the trpl gene which provides a selection marker for a mutant strain of yeast lacking the ability to grow in tryptophan, for example ATCC No. 44076 or PEP4-1 (Jones, Genetics, 85: 12 (1977)). The presence of the trpl lesion as a characteristic of the yeast host cell genome then provides an effective environment for detecting transformation by growth in the absence of tryptophan.

Suitable promoting sequences in yeast vectors, include the promoters for 3-phosphoglycerate kinase (Hitzeman, et al., J. Biol. Chem., 255:12073 (1980)) or other glycolytic enzymes (Hess, et al, J. Adv. Enzyme Reg., 7: 149 (1968); Holland, et al, Biochemistry, 17: 4900 (1978)), such as enolase, glyceraldehyde-3-phosphate dehydrogenase, hexoki-nase, pyruvate decarboxylase, phosphofructokinase, glucose-6-phosphate isomerase, 3 - phosphoglycerate mutase, pyruvate kinase, triosephosphate isomerase, phosphoglucose isomerase, and glucokinase. In constrcting suitable expression plasmids, the termination sequences associated with these genes are also ligated into the expression vector 3' of the sequence desired to be expressed to provide polyadenylation of the mRNA and termination. Other promoters, which have the additional advantage of transcription controlled by growth conditions are the promoter regions for alcohol dehydrogenase 2, isocytochrome C, acid phosphatase, degradative enzymes associated with nitrogen metabolism, and the aforementioned glyceraldehyde -3- phosphate dehydrogenase, and enzymes responsible for maltose and galactose utilization (Holland, ibid.). Any plasmid vector containing yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propogation of vertebrate cells in culture (tissue culture) has become a routine procedure in recent years [Tissue Culture, Academic Press, Kruse and Patterson, editors (1973)]. Examples of such useful host cell lines are VERO and HeLa cells, Chinese hamster ovary (CHO) cell lines, and W138, BHK, COS-7 and MDCK cell lines. Expression vectors for such cells ordinarily include (if necessary) an origin of replication, a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promoters are derived from polyoma, Adenovirus 2, and most frequently Simian Virus 40 (SV40). The early and late promoters of SV40 virus are particularly useful because both are obtained easily from the virus as a fragment which also contains the SV40 viral origin of replication (Fiers, et al., Nature, 273: 113 (1978). Smaller or larger SV40 fragments may also be used, provided there is included the approximately 250 bp sequence extending from the Hind I site toward the Bgl I site located in the viral origin of replication. Further, it is also possible, and often desirable, to utilize promoter or control sequences normally associated with the desired gene sequence, provided such control sequences are compatible with the host cell systems.

An origin of replication may be provided either by construction of the vector to include an exogenous origin, such as may be derived from SV40 or other viral (e.g. Polyoma, Adeno, VSV, BPV) source, or may be provided by the host cell chromosomal replication mechanism. If the vector is integrated into the host cell chromosome, the latter is often sufficient.

In selecting a preferred host cell for transfection by the vectors of the invention which comprise DNA sequences encoding both t-PA and DHFR protein, it is appropriate to select the host according to the type of DHFR protein employed. If wild type DHFR protein is employed, it is preferable to select a host cell which is deficient in DHFR, thus permitting the use of the DHFR coding sequence as a marker for successful transfection in selective medium which lacks hypoxanthine, glycine, and thymidine. An appropriate host cell in this case is the Chinese hamster ovary (CHO) cell line deficient in DHFR activity, prepared and propagated as described by Urlaub and Chasin, Proc. Natl. Acad. Sci. (USA) 77: 4216 (1980).

On the other hand, if DHFR protein with low binding affinity for MTX is used as the controlling sequence, it is not necessary to use DHFR deficient cells. Because the mutant DHFR is resistant to methotrexate, MTX containing media can be used as a means of selection provided that the host cells are themselves methotrexate sensitive. Most eukaryotic cells which are capable of absorbing MTX appear to be methotrexate sensitive. One such useful cell line is a CHO line, CHO-K1 ATCC No. CCL 61.

Examples which are set forth hereinbelow describe use of E. coli using the lac and trp promoter system and use of CHO cells as host cells, and expression vectors which include the SV40 origin of replication as a promoter. However, it would be well within the skill of the art to use analogous techniques to construct expression vectors for expression of desired protein sequences in alternative prokaryotic or eukaryotic host cell cultures.

Satisfactory amounts of human t-PA are produced by cell cultures, however, later refinements using a secondary coding sequence serve to enhance production levels even further. The secondary coding sequence encodes dihydrofolate reductase (DHFR) which is affected by an externally controlled parameter, such as methotrexate, thus permitting control of expression by control of the methotrexate (MTX) concentration.

### C. Methods employed

If cells without formidable cell membrane barriers are used as host cells, transfection is carried out by the calcium phosphate precipitation method as described by Graham and Van der Eb, Virology, 52: 456 (1973). However, other methods for introducing DNA into cells such as by nuclear injection or by protoplast fusion may also be used.

If prokaryotic cells or cells which contain substantial cell wall constructions are used, the preferred method of transfection is calcium treatment using calcium chloride as described by Cohen, F N. et al Proc. Natl. Acad. Sci. (USA), 69: 2110 (1972).

Construction of suitable vectors containing the desired coding and control sequences employ standard ligation techniques. Isolated plasmids or DNA fragments are cleaved, tailored, and religated in the form desired to from the plasmids required.

Cleavage is performed by treating with restriction enzyme (or enzymes) in suitable buffer. In general, about 1 pg plasmid or DNA fragments is used with about 1 unit of enzyme in about 20 J.l1 of buffer solution. (Appropriate buffers and substrate amounts for particular restriction enzymes are specified by the manufacturer.) Incubation time of about 1 hour at 37°C are workable. After incubations, protein is removed by extraction with phenol and chloroform, and the nucleic acid is recovered from the aqueous fraction by precipitation with ethanol.

If blunt ends are required, the preparation is treated for 15 minutes at 15°C with 10 units of Polymerase I (Klenow), phenolchloroform extracted, and ethanol precipitated.

Size separation of the cleaved fragments is performed using 6 percent polyacrylamide gel described by Goeddel, D., et al, Nucleic Acids Res., 8: 4057 (1980) incorporated herein by reference.

For ligation approximately equimolar amounts of the desired components, suitably end tailored to provide correct matching are treated with about 10 units T4 DNA ligase per 0.5 pg DNA. (When cleaved vectors are used as components, it may be useful to prevent religation of the cleaved vector by pretreatment with bacterial alkaline phosphatase.)

For analysis to confirm correct sequences in plasmids constructed, the ligation mixtures are used to transform E. coli K12 strain 294 (ATCC 31446), and successful transformants selected by ampicillin or tetracycline resistance where appropriate. Plasmids from the transformants are prepared, analyzed by restriction and/or sequenced by the method of Messing, et al, Nucleic Acids Res., 9:309 (1981) or by the method of Maxam, et al, Methods in Enzymology, 65:499 (1980).

Amplification of DHFR protein coding sequences is effected by growing host cell cultures in the presence of approximately 20-500,000 nM concentrations of methotrexate, a competitive inhibitor of DHFR activity. The effective range of concentration is highly dependent, of course, upon the nature of the DH FR gene, protein and the characteristics of the host. Clearly, generally defined upper and lower limits cannot be ascertained. Suitable concentrations of other folic acid analogs or other compounds which inhibit DHFR could also be used. MTX itself is, however, convenient, readily available and effective.

### D. General description of preferred embodiments

Human tissue plasminogen activator was obtained according to the following protocol:
1. Human melanoma cells actively producing tissue plasminogen activator were cultured to confluency.
2. Cell pellets from such cell cultures were extracted in the presence of ribonuclease inhibitors to isolate all cytoplasmic RNA.
3. An oligo-dT column isolated the total messenger (mRNA) in polyadenylated form. This mRNA was size fractionated using acid-urea agarose gel electrophoresis.
4. The gel fraction containing tissue plasminogen activator specific RNA was identified in the following manner: The RNA from each of the gel fractions was translated in a rabbit reticulocyte lysate in vitro system supplemented with dog pancreas microsomes. The resulting translation products were then immunoprecipitated with human tissue plasminogen activator specific IgG antibody.
5. The appropriate RNA (21 to 24S) was converted to corresponding single stranded complementary DNA (cDNA) from which was produced double stranded cDNA. After poly-dC tailing, it was inserted into a vector, such as a plasmid bearing one or more phenotypic markers.
6. The thus prepared vectors were used to transform bacterial cells providing a cloned cDNA library A pool of radiolabelled synthetic deoxy oligonucleotides complementary to codons for known amino acid sequences in t-PA, such as, for example the pool of 8 14-mers, 5'-dTC( 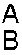 )CA( 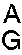 )TA( 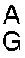 )TCCCA-3' (complementary to sequences coding for the known-see infra-amino acid sequence: tryptophanglutamic acid-tryosine-cysteine-aspartic acid (WE-Y-C-D) was prepared and used to probe the colony library.
7. From the positive cDNA clones plasmid DNA was isolated and sequenced.
8. The sequenced DNA encoding t-PA was then tailored in vitrofor insertion into an appropriate expression vehicle which was used to transform an appropriate host cell, which was, in turn, permitted to grow in a culture and to produce the desired human tissue plasminogen activator.
9. Human tissue plasminogen activator thus produced has ca. 251 amino acids in its enzymatic serine protease portion and a "kringle" containing sequence upstream therefrom which is presently believed to be responsible for fibrin binding. The mature protein plus its signal presequence, totals 562 amino acids.

The foregoing procedure, in itself, is successful in producing pure t-PA. Methods of the invention employing an additional coding sequence sensitive to methotrexate permit the production in host cell cultures of antigenically active t-PA protein in amounts greater than 0.1 pg per cell per day. With suitable application of amplifying conditions, amounts greater than 20 pg per cell per day can be obtained. Stated in alternative terms, gene expression levels resulting in production of more than 9X10⁻⁶ Plough units per cell per day, or, with suitable amplification, more than 18X10⁻⁴ Plough units of t-PA activity are achieved.

Advantage is taken in this aspect of the invention of methotrexate as a drug which, while normally fatal to cells capable of its uptake, permits cells to grow in the presence of controlled levels of MTX by amplification of the gene coding for the DHFR coding sequence (Schimke, Robert T. et al, Science, 202: 1051 (1978); Biedler, J. L. et al. Cancer Res. 32:153 (1972); Chang, S. E., et al, Cell, 7: 391 (1976)).

Of importance to this aspect of the invention is the showing that amplification of the gene for DHFR may cause amplification of associated sequences which code for other proteins. This appears to be the case when the associated protein is hepatitis B surface antigen (HBsAg) (Christman, J. et al. Proc. NatL Acad. Sci. 79: 1815 (1982)); the E. coli ^{p}rotein XGPRT (Ringold, Gordon, et al, J. Molec. and Appl. Gen., 1: 165 (1981 )); and an endogenous sequence from a DHFR/SV40 plasmid combination (Kaufman, R. F. et al, J. Molec. Biol., 159: 601 (1982)).

Other mechanisms for conferring methotrexate resistance include diminution of the binding affinity of the DHFR protein, so that it is less susceptible to methotrexate (Flintoff, W. F., et al, Somat. Cell Genet., 2 : 245 (1976)) but in this instance, amplification appears to occurs as well.

It would appear that the genes both for wild type DHFR and for DHFR which is resistant to MTX by virtue of its own decreased binding capacity are amplified by the presence of MTX. Hence, in principle, this aspect of the invention herein concerns using the impact of DHFR sequence amplification on associated protein coding sequences to provide a control mechanism which permits enhanced expression levels of t-PA sequences in the presence of MTX, or by virtue of prior treatment of transformed cells with MTX.

### E. Examples

The following examples are intended to illustrate but not to limit the invention. In the examples here are E. coli host culture and a CHO cell line suitable for the type of DHFR protein coding sequence to be introduced were employed as host cell cultures. However, other eukaryotic and prokaryotic cells are suitable forthe method of the invention as well. E.1 Expression of the human t-PA gene in E. coli

### E.1.A Figure legends

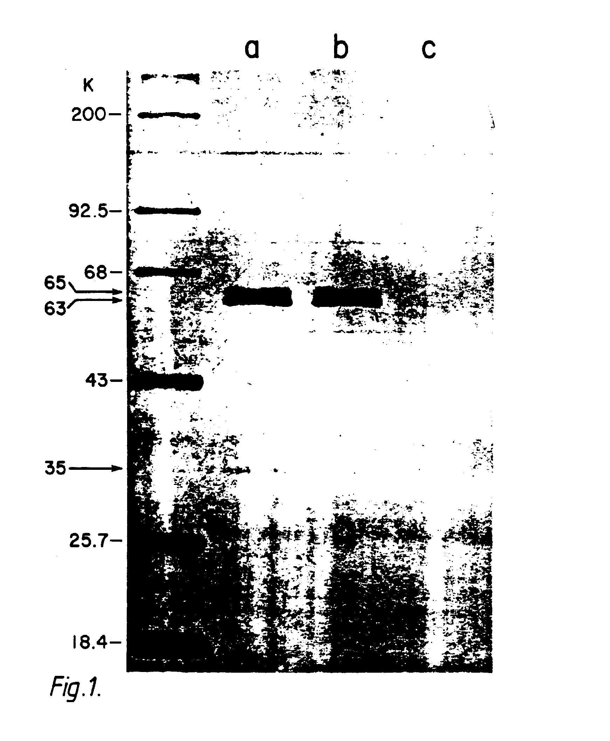

Figure 1 is an autoradiogram of a 10 percent SDS PAGE displaying the immunoprecipitated [³⁵S]-methionine labelled protein(s) secreted from numan melanoma cells during a 3 hour pulse in vivo, in the presence (lane b) or absence (lane a) of the protease inhibitor aprotinin. After immunoprecipitation with tissue plasminogen activator specific IgG, three bands were observed (lane a) having molecular weights of approximately 65,000, 63,000 and 35,000. In the presence of the protease inhibitor, however, no 35,000 molecular weight species is observed. No products are immunoprecipitated where preimmune serum is used (lane c). The migrations and molecular weights of ¹⁴C-labelled protein standards are shown to the left of lane a.

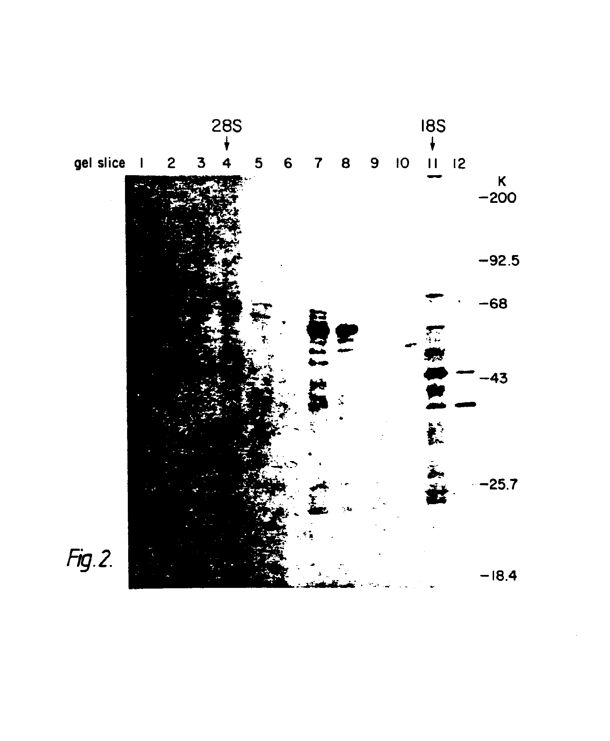

Figure 2 depicts the gel electrophoresis of the immunoprecipitated translation products of RNA fractions isolated from an acid urea agarose gel. A major band was observed in fraction numbers 7 and 8 after translation in the presence of dog pancreas microsomes followed by immune precipitation with tissue plasminogen activator specific IgG. This band has a molecular weight of approximately 63,000 daltons. The size of the mRNA migrating in fractions 7 and 8 is approximately 21 to 24S. The positions of ribosomal RNA markers which were determined after electrophoresis on the RNA urea gel and visualized by ethidium bromide staining are labelled above the appropriate gel lanes.

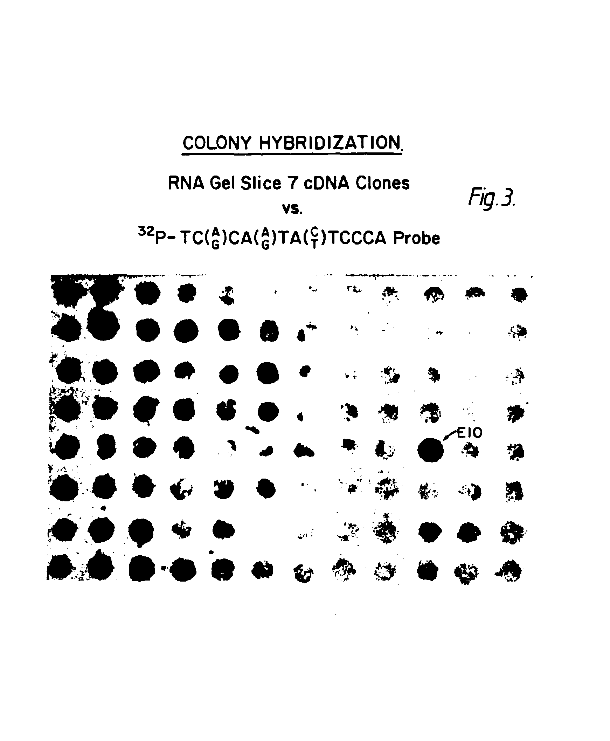

Figure 3 displays the hybridization pattern of 96 colonies with ³²P- -dTC( 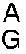 )CA( 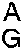 )TA( 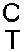 )TCCCA (W-E-Y-C-D) probe. 96 individual transformants were grown in a microtiter plate, replica plated and grown on a nitrocellulose membrane. The colonies were then lysed, bacterial DNA fixed and the filters were hybridized with the ³²P-14-mer (W-E-Y-C-D) probes. The filters were washed to remove nonhybridized probe and exposed to X-ray film. This autoradiogram is representative of the patterns obtained with 48 individual filters (4600 independent colonies). An example of a positive tissue plasminogen activator cDNA clone on filter number 25 is labelled as E10 (arrow).

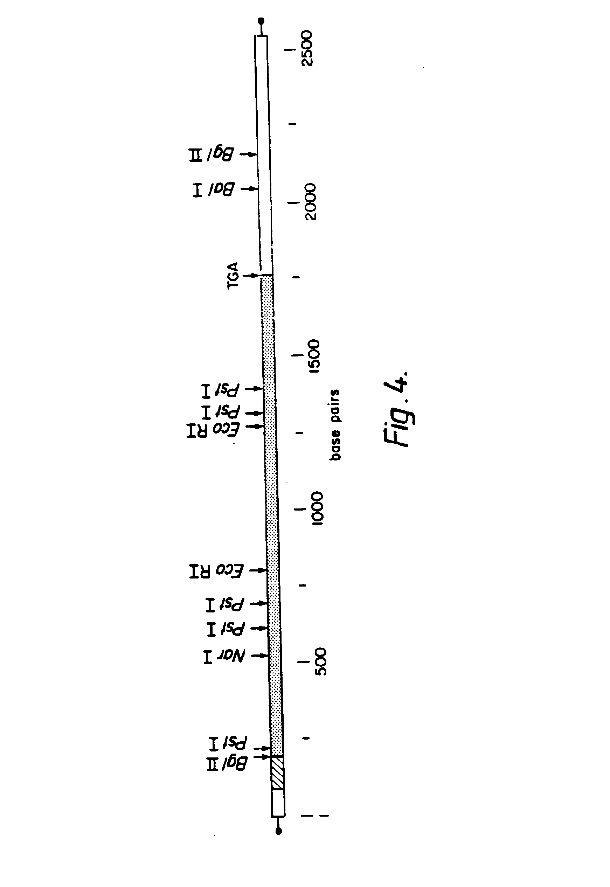

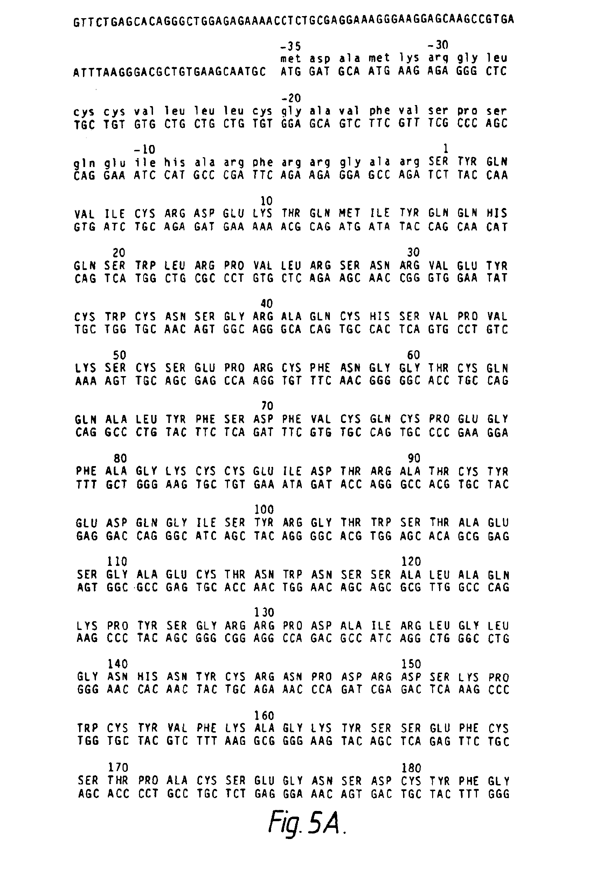

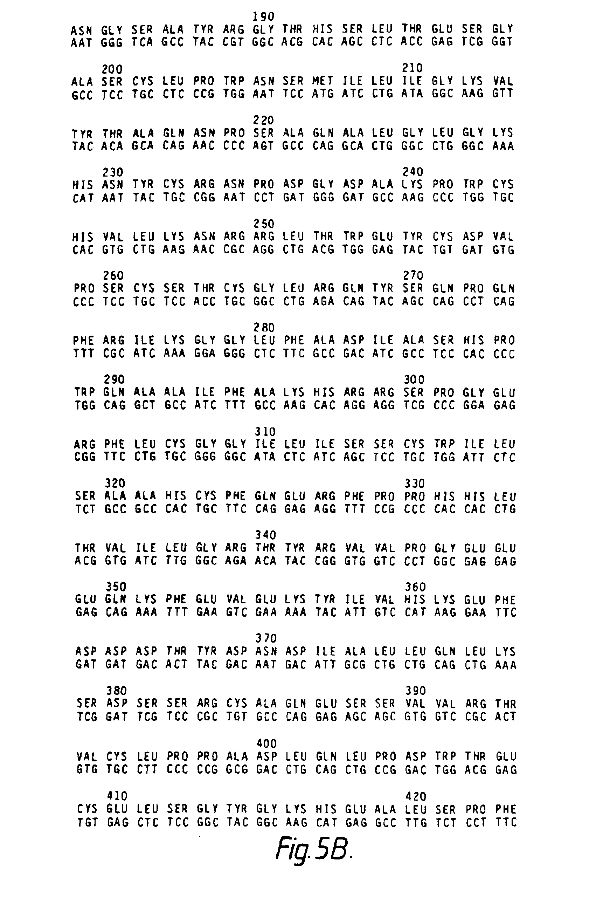

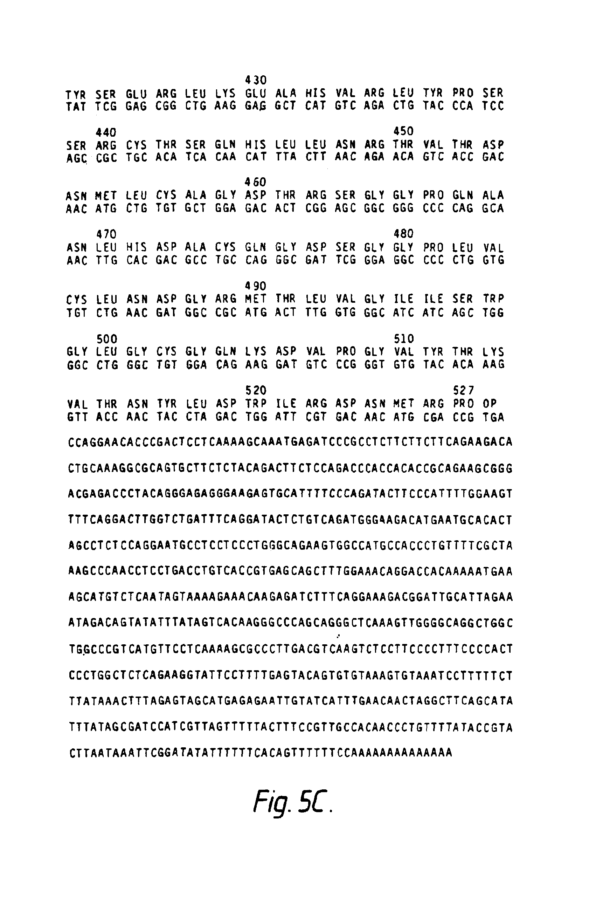

Figure 4 is a restriction endonuclease map of the full length human tissue plasminogen activator _{C}DNA. The number and size of fragments produced by restriction endonuclease cleavage was estimated by electrophoresis through 6 percent acrylamide gels. Positions of sites were confirmed by nucleic acid sequence (presented in Figure 5). The coding region of the largest open reading frame is boxed and the hatched region represents the putative signal peptide sequence, while the stipled region represents the putative mature tissue plasminogen activator sequence (527 amino acids). The 5' end of the mRNA is to the left while the 3' end is to the right.
Figures 5A, 5B, and 5C illustrate the nucleotide sequence and deduced amino acid sequence of the full length human tissue plasminogen activator cDNA. The 35 amino acids (-35 to -1 ) preceding the mature sequence is depicted as an uninterrupted sequence. It is believed that this 35-amino acid sequence is comprised of a hydrophilic "pro" sequence, preceding serine (+1 ) of the mature protein, of about 12 to 15 amino acids, in turn preceded by a "conventional" hydrophobic signal (extending 5' to -35). This type of pre-pro structure on secreted proteins has been described previously, e.g. with preproalbumin. Assuming this theory, all of the secrete tissue plasminogen activator molecules will start with the serine (+1) as the amino-terminus. A second theory is that the hydrophilic sequence could be involved with the function of tissue plasminogen activator in a manner analogous to that observed with plasminogen where a peptide of 10,000 daltons can be cleaved from the amino terminal portion of native plasminogen (Glu-plasminogen, named for the amino terminal residue), resulting in a smaller molecule, with a new amino terminus, designated Lys-plasminogen. Lys-plasminogen is more easily activated to plasmin, and also has a greater affinity for fibrin than Glu plasminogen. Plasmin has been shown to catalyze the conversion of Glu- or Lys-plasminogen. This type of control mechanism results in a "positive feedback" mechanism. The first amounts of plasmin formed, beside degrading fibrin, also result in the generation of plasminogen molecules which are more easily activated, and also bind tighter to their substrate, than native plasminogen. The result is a faster degradation of fibrin. The hydrophilic peptide of tissue plasminogen activator could be involved in a similar mechanism, its cleavage resulting in modified binding of the enzyme to fibrin. In any event the 35 amino acid sequence is considered a presequence of the mature protein.

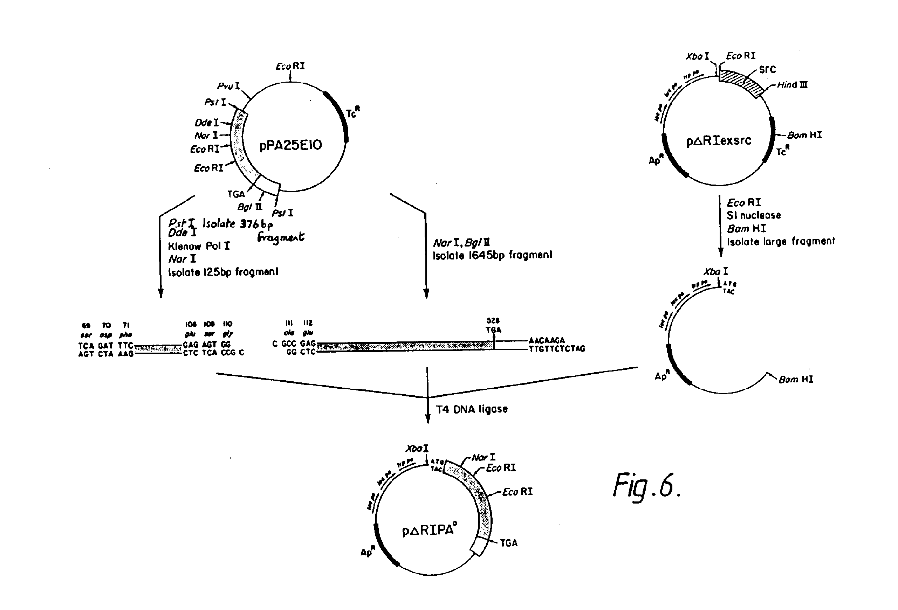

Figure 6 is a schematic diagram of the construction of a tissue plasminogen activator expression plasmid pΔRIPA°. The starting plasmid pPA25E10 was first digested with Pstl to isolate a 376 bp. fragment that was then digested as shown in the figure.

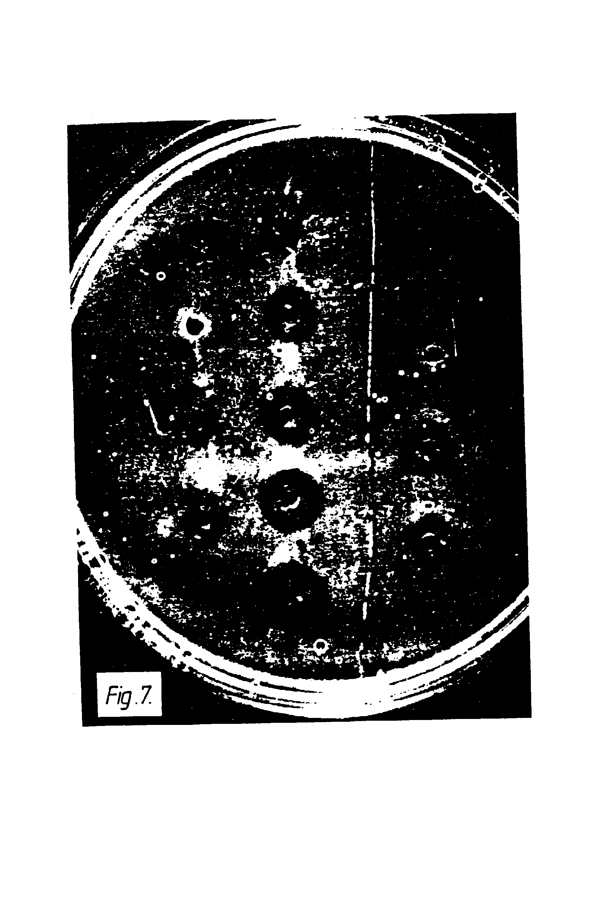

Figure 7 shows the result of a fibrin plate assay for fibrinolytic activity of the expression product obtained via p_{O}RIPA° in transformed cells.

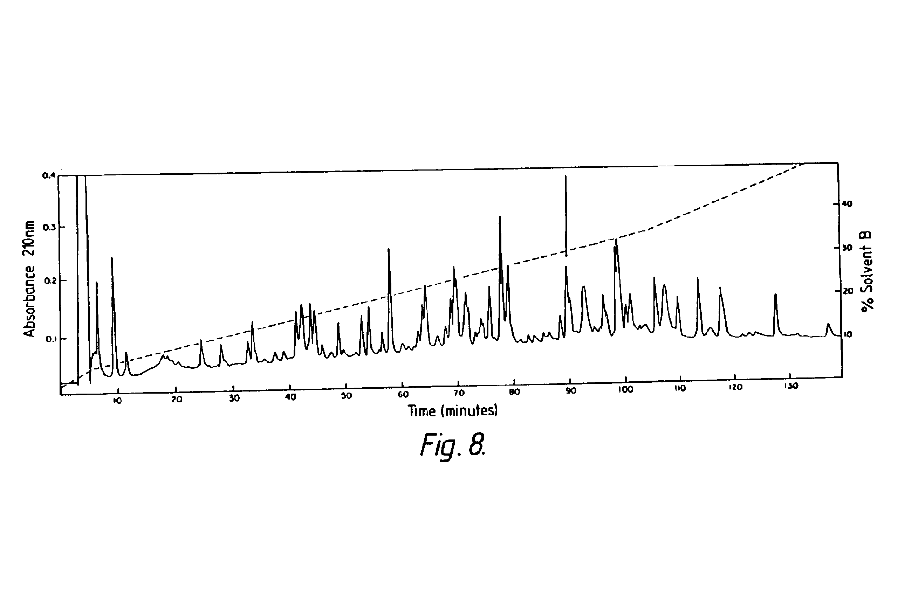

Figure 8 is an HPLC trace of peptides from tissue plasminogen activator (hereof) trypsin digest (Absorbance at 210 nm). The arrow identifies the peak corresponding to the peptide used to design the nucleotide probe used with the colony library. The peptide represented by this peak was found to have the entire sequence: L—T—W—E—Y—C—D—V—P—S—C—S—T—C—G—L. The other major peaks likewise were sequenced and found to confirm the correct amino sequence of human tissue plasminogen activator. The peptide one letter code referring to amino acid designations is as follows: 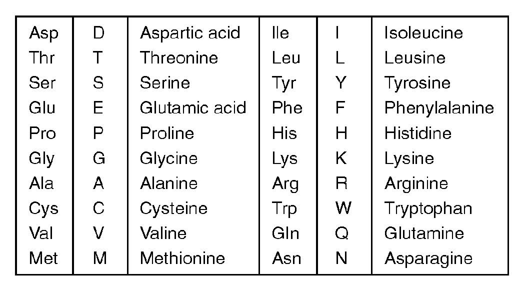

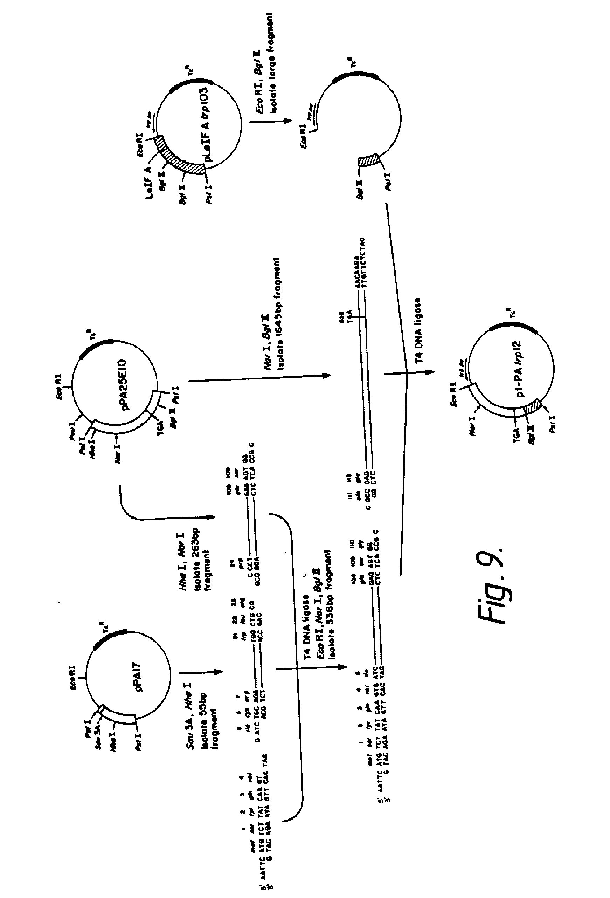

Figure 9 depicts the construction of a plasmid coding for the direct expression of mature human tissue plasminogen activator in E. coli. 50 pg of plasmid pPA17 was digested with Sau 3AI, Hinc∥ and Hhal and electrophoresed on a 6 percent polyacrylamide gel.

Approximately 0.5 µg of the 55 bp Sau3Al-Hhal fragment was recovered. Similarly, approximately 3 pg of the 263 bp Hhal-Narl fragment was purified from 80 µg of clone pPA25E10 by first isolating a 300 bp Pstl-Narl fragment and then digesting this fragment with Hhal. All digests were performed at 37°C for 1 hour and the reaction products resolved and electroeluted from 6 percent polyacrylamide gels. The two indicated deoxyoligonucleotides 5′ dAATTCATGTCTTATCAAGT(I) and 5′ GATCACTTGATAAGACATG (II) were synthesized by the solid phase phosphotriester method (51). 100 pmole of oligonucleotide ∥ was phosphorylated in a 30 µl reaction mixture containing 60 mM Tris (pH 8), 10 mM MgCl₂, 15 mM [β-mercaptoethanol and 50 ₄Ci [γ³²P] ATP (Amersham 5,000 Cimmol⁻¹) 12 units of T4 polynucleotide kinase were added and the reaction allowed to proceed at 37°C for 15 min. One µl of 10 mM ATP and 12 units of T4 kinase were then added and the reaction allowed to proceed for an additional 30 min. After phenol/CHCl₃ extraction, the phosphorylated oligomer ∥ and the 5' hydroxyl oligomer I were combined with 0.5 µg of the eluted 55 bp Sau3A|-Hha| fragment and 2 pg of the 263 bp Hhal-Narl fragment and ethanol precipitated. These fragments were ligated at room temperature for 4 hours in 60 µl of 20 mM Tris-HCI (pH 7.5), 10 mM MgCl₂, 10 mM dithiothreitol, 0.5 mM ATP and 1000 units of T4 DNA ligase. The mixture was digested for 1 hour with 48 units of Narl, i20 units of EcoRl and 40 units of Bgl∥ (to eliminate polymerization through ligation of cohesive Sau3AI termini) and electrophoresed on a 6 percent gel. The 338 bp product (approximately 0.1 µg) was recovered by electroelution. The remainder of the t-PA coding sequences (amino acids 111-528) were isolated on a 1645 bp fragment by digesting plasmid pPA25E10 with Narl and Bg/∥. The plasmid pLeIFAtrp103 is a derivative of the plasmid pLelFA25 (52) in which the EcoRl site distal to the LelF A gene has been removed (53). Three µg of pLeIFAtrp103 were digested with 20 units of EcoRl and 20 units of Bglll for 90 min. at 37°C, electrophoresed on 6 percent polyacrylamide gel and the large (~4,200 bp) vector fragment was recovered by electroelution. For the final construction, 80 ng of EcoRl -BgA/ll pLeIFAtrp103 was ligated with 100 ng of the 1645 bp Narl -BgA/ll fragment and 20 ng of the 338 bp Eco-RI-Nari fragment for 10 hours at room temperature. This ligation mixture was used to transform E. coli K-12 Strain 294. Plasmid DNA was prepared from 38 of these transformants and digested with EcoRl. Ten of these plasmids contained the desired 600 bp and 472 bp EcoRl fragments. DNA sequence analysis verified that one of these plasmids (pt-PAtrpl2) had the desired nucleotide sequence at the junctions between the trp promoter, synthetic DNA and cDNA.

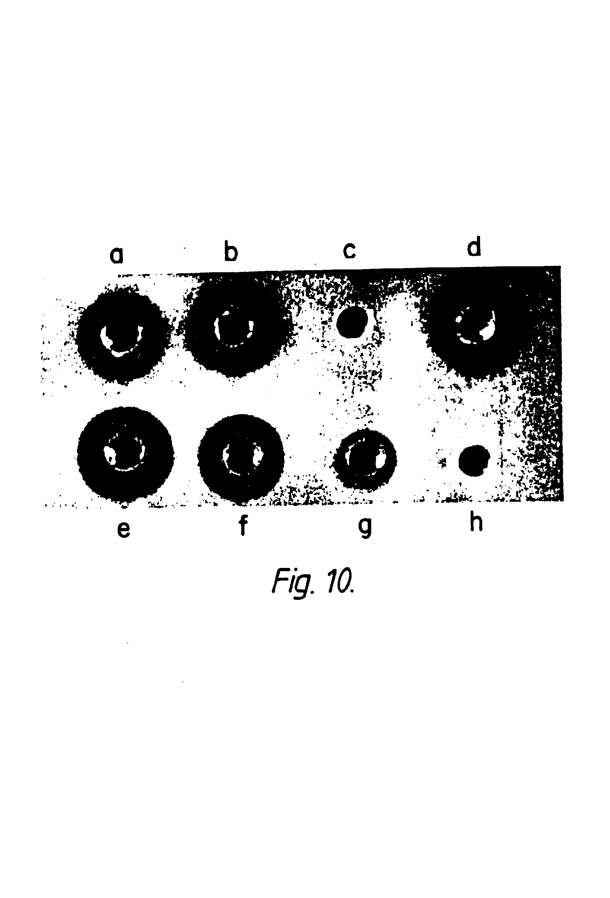

Figure 10 shows the result of a fibrin plate assay for fibrinolytic activity of a tissue plasminogen activator expression product hereof. An overnight culture of E. coli W31101/pt-PAtrpl2 in Luria broth containing 5 µg ml-¹ tetracycline was diluted 1:100 in M9 medium containing 0.2 percent glucose, 0.5 percent casamino acids and 5 ^{p}g ml-¹ tetracyline. The cells were grown at 37°C to an A₅₅₀ of 0.2 and indole acrylic acid was added to a final concentration of 20 pg/ml. Samples were collected by centrifugation at A₅₅₀=0.5-0.6 (~2×10⁸ cells ml⁻¹) and immediately frozen. The cell pellets were suspended in 6M guanidine hydrochloride at 5×10⁸ cells/ml, sonicated for 10 sec, incubated at 24°C for 30 min and then dialyzed for 4 hrs against 25 mM Tris-HCI pH 8.0,250 mM NaCl, 0.25 mM EDTA and 0.01 percent Tween 80^{*} After dialysis the samples were centrifuged at 13,000 Xg for 2 min and 10 pₗ of the supernatants analyzed for tissue plasminogen activator activity. Following the procedure of Granelli-Piperno and Reich (87), the plate was incubated for 3.5 hours at 37°C and lysis zones measured. Quantification was obtained by comparison to dilutions of a purified melanoma tissue plasminogen activator solution.

### ^{*} Trade Mark

### E.1.B Source of tissue plasminogen activator mRNA

Human melanoma cells (Bowes) were used. The melanoma cells were cultured to confluent monolayers in 100 ml Earles Minimal Essential Media supplemented with sodium bicarbonate (0.12 percent final concentration), 2 mM glutamine and 10 percent heat-inactivated fetal calf serum. To confirm that the melanoma cells were actively producing human tissue plasminogen activator, human melanoma cells were cultured to confluency in a 24 well microtiter dish. Either in the presence or absence of 0.33 µM the protease inhibitor aprotinin, the cells were washed once with phosphate buffered saline and 0.3 ml of serum free methionine free medium was added. 75 pCi of [³⁵S]-methionine was added and the cells were labelled at 37°C for 3 hours. At the end of the 3 hour labelling period the media was removed from the cells and treated with either tissue plasminogen activator specific IgG or pre-immune serum for immunoprecipitation (54). The immunoprecipitated products were displayed by electrophoresis on a 10 percent SDS-acrylamide gel. The slab gel was fixed, dried and subjected to fluorography.

### E.1.C Messenger RNA isolation and size fractionation

Total RNA from melanoma cell cultures was extracted essentially as reported by Ward et al. (55). Cells were pelleted by centrifugation and then resuspended in 10 mM NaCl, 10 mM Tris-HCI pH 7.5, 1.5 mM MgCl₂. Cells were lysed by the addition of NP-40 (1 percent final concentration), and nuclei were pelleted by centrifugation. The supernatant contained the total RNA which was further purified by multiple phenol and chloroform extractions. The aqueous phase was made 0.2 M in NaCI and then total RNA was precipitated by the addition of two volumes of ethanol. Oligo-dT cellulose chromatography was utilized to purify mRNA from the total RNA preparations (54). Typical yields from 10 grams of cultured melanoma cells were 5 to 10 milligrams of total RNA and 50-200 micrograms of Poly(A) plus mRNA.

Fractionation of PolyA+ mRNA (200 pg) (56) was performed by electrophoresis through urea-agarose gels. The slab agarose gel (57, 58) was composed of 1.75 percent agarose, 0.025 M sodium citrate, pH 3.8 and 6 M urea. Electrophoresis was performed for 7 hours at 25 milliamps and 4°C. The gel was then fractionated with a razor blade. The individual slices were melted at 70°C and extracted twice with phenol and once with chloroform. Fractions were then ethanol precipitated and subsequently assayed by in vitrotranslation in a rabbit reticulocyte lysate system, Bethes-da Research Lab. (59, 60), supplemented with dog pancreas microsomes as follows: Translations were performed using 25 µCi of [³⁵S] methionine and 500 nanograms of each gel slice RNA in a final volume of 30 µl containing 25 mM HEPES, 48.3 mM potassium chloride, 10 mM creatine phosphate. 19 amino acids at 50 mM each, 1.1 mM magnesium chloride 16.6 mM EDTA, 0.16 mM dithiothreitol 8.3 mM hemin, 16.6 pg/ml creatine kinase, 0.33 mM calcium chloride, 0.66 mM EGTA, 23.3 mM sodium chloride.

Incubations were carried out at 30°C for 90 minutes. Dog pancreas microsomal membranes prepared from rough microsomes using EDTA for removal of the ribosomes (61 ) were treated with nuclease as described (62) and were present in the translation mixture at a final concentration of 7 A₂₆0 units/ml. Translation products or immunoprecipitated translation products were analyzed by electrophoresis on 10 percent polyacrylamide gels in sodium dodecyl sulfate as previously described (63). The unstained slab gels were fixed, dried and subjected to fluorography (64).

The resulting translation products from each gel fraction were immunoprecipitated with rabbit anti-human tissue plasminogen activator specific IgG. One major immunoprecipitated polypeptide band was observed in the translation of RNA fraction numbers 7 and 8 (migration of 21 to 24S) having molecular weight of approximately 63,000 daltons. This band was not observed when preimmune IgG was used for immunoprecipitation which suggested these polypeptides were tissue plasminogen activator specific.

E.1.D. Preparation of a colony library containing tissue plasminogen activator sequences Five µg of gelfractionated mRNA (gel slice 7 mRNA) was used for the preparation of double stranded cDNA by standard procedures (52, 65, 66). The cDNA was size fractionated on a 6 percent polyacrylamide gel. The cDNA greater than 350 base pairs in length (125 ng) was electroeluted. 30 ng of cDNA was extended with deoxy(C) residues using terminal deoxynucleotidyl transferase (67) and annealed with 300 ng of the plasmid pBR322 (68) which had been similarly tailed with deoxy(G) residues at the Pst I site (67). The annealed mixture was then transformed into E. coli K12 strain 294 (ATCC No. 31446). Approximately 4,600 transformants were obtained.

### E.1.E Preparation of DNA probe

Purified human tissue plasminogen activator was obtained according to the procedure of disclosed references (19, 20).

The molecule was scanned in order to locate regions best suited for making synthetic probes, as follows:
To make the proteins susceptible to digestion by trypsin it was reduced and carboxymethylated. A 2 mg sample of tissue plasminogen activator was first dialyzed against 0.01 percent Tween 80 over night at room temperature. The lyophilized protein was then dissolved in 10 ml of 0.56 M Tris-HCI buffer (pH 8.6), 8 molar in urea and 5 mM EDTA. The disulfide bonds were reduced by the addition of 0.1 ml of β-mercaptoethanol. This reaction was carried out under nitrogen for 2 hours at 45°C. The reduced disulfides were alkylated to the carboxymethyl derivative by the addition of 1.0 ml. of 1.4 M iodoacetic acid in 1 N NaOH. After 20 min at room temperature the reaction was stopped by dialysis against 0.01 percent Tween 80 for 18 hours at room temperature and lyophilized.

The resulting lyophilized carboxymethylated protein was redissolved in 3 ml of 0.1 M sodium phosphate buffer (pH 7.5). Trypsin (TPCK) was added (1 to 50 ratio) and digested at 37°C. Aliquots (0.1 ml) were taken at 3 hours, 6 hours, and 12 hr. A second addition of trypsin was made at 12 hr. The reaction was stopped for 24 hr by freezing the sample until it could be injected on the HPLC. The progress of the digestion was determined by SDS gels on the aliquots. All gels were blank except for a faint band on the 3 hour aliquot. This indicated that the 24 hour digestion was complete and no large peptides remained.

A sample (ca. 0.5 ml) was injected into a high resolution Altex C-8 ultrasphere 5 µ column with two runs. A gradient of acetonitrile was made gradual (1 percent to 5 percent in 5 min, 5 percent to 35 percent in 100 min, 35-50 percent in 30 min). In one of the two preparative runs, the eluant was monitored at two wavelengths (210 nm and 280 nm). The ratio of the two wavelength absorptions was used to indicate the tryptophan containing peptides.

The peptide peaks most likely to contain tryptophan, or that were believed useful for other reasons, were sequenced first. This enabled the determination of the sequence around most of the tryptophans. After sequencing about 25 of the best possible peptide peaks, all the sequence data that could be aligned was pooled to obtain a preliminary model of the primary structure of tissue plasminogen activator. From this data and model, several possible probes were located.

### E.1. F. Identification of bacterial clones containing tissue plasminogen activator cDNA sequences

The colonies were individually inoculated into wells of microtiter plates containing LB (93)+5 µg/ml tetracycline and stored at -20°C after addition of DMSO to 7 percent. Two copies of the colony library were grown up on nitrocellulose filters and the DNA from each colony fixed to the filter by the Grunstein Hogness procedure (69).

The³²P-labelled-TC(^{A}G)CA(^{A}G)TA(G)TCCCA probe was prepared (from the synthetic oligomer) (W-E-Y-C-D) 14-mer pool as described above. Filters containing 4,600 transformants were prehybridized for 2 hours at room temperature in 50 mm sodium phosphate DH 6.8. 5X SSC (80), 150 ug/ml sonicated salmon sperm DNA, 5XDenhardt's solution (85) 10 percent formamide and then hybridized with 50×10⁶ counts per minute of the labelled probe in the same solution. After an overnight incubation at room temperature, the filters were washed 3 times at room temperature in 6xSCC, 0.1 percent SDS for 30 minutes, once in 2xSSC and then exposed to Kodak XR-5 x-ray film with Dupont Lightning Plus intensifying screens for 16 hours.

Plasmid DNA was isolated by a rapid method (71) from all colonies showing a positive hybridization reaction. The cDNA inserts from these clones were then sequenced after subcloning fragments into the M13 vector mp7 (73) and by the Maxam Gilbert chemical procedure (74). Figure 3 displays filter number 25 showing in the hybridization pattern a positive tissue plasminogen activator clone. The cDNA insert in clone 25E 10 was demonstrated to be the DNA coding for tissue plasminogen activator by comparing its amino acid sequence with peptide sequence (See Supra) obtained from purified tissue plasminogen activator and by its expression product produced in E. colias described in more detail infra. The cDNA insert of clone 25E1 (plasmid pPA25E10) was 2304 base pairs in length with the longest open reading frame encoding a protein of 508 amino acids (MW of 56,756) and containing a 772 bp 3' untranslated region. This cDNA clone lacked the N-terminal coding sequences.

### E.1.G Direct expression of a human tissue plasminogen activator clone in E. coli

With reference to Figure 6, 50 pg of pPA25E10 (supra) were digested with Pst I and the 376 bp fragment isolated by electrophoresis on a 6 percent polyacrylamide gel. Approximately 3 µg of this fragment was isolated from the gel by electroeluting, digested with 30 units of Dde I for 1 hr at 37°C phenol and chloroform extracted, and ethanol precipitated. The resulting Dde I sticky ends were extended to blunt ends by adding 5 units of DNA polymerase I (Klenow fragment) and 0.1 mM each of dATP, dCTP, dGTP, dTTP to the reaction mixture and incubating at 4°C for 8 hours. After extraction with phenol and chloroform, the DNA was digested with 15 units of Nar I for 2 hours and the reaction mixture electrophoresed on a 6 percent polyacrylamide gel. Approximately 0.5 µg of the desired 125 bp blunt end Nar I fragment was recovered. This fragment codes for amino acids number 69 through 110 of the mature full length tissue plasminogen activator protein.

For isolation of the 1645 bp Nar lBgl it fragment, 30 µg of pPA25E10 were digested with 30 units of Nar I and 35 units of Bgl I I for 2 hours at 37°C and the reaction mixture electrophoresed on a 6 percent polyacrylamide gel. Approximately 6 µg of the desired 1645 bp Nar I Bgl ll fragment were recovered.

The plasmid pAR1 exSRC is a derivative of the plasmid pSRCe×16 (79) in which the EcoRl sites proximal to the ^{*}Trade mark trp promoter and distal to the SRC gene have been removed by repair with DNA polymerase 1(28), and the self-complementary oligodeoxynucleotide AATTATGAATTCAT (synthesized by the phosphotriester method (75) was inserted into the remaining Eco RI site immediately adjacent to the Xba I site. 20µg of pAR1 exSRC were digested to completion with Eco Rl , phenol and chloroform extracted, and ethanol precipitated. The plasmid was then digested with 100 units of nuclease S1 at 16°C for 30 minutes in 25 mM sodium acetate (pH 4.6), 1 mM ZnCl₂ and 0.3 M NaCl to create a blunt end with the sequence ATG. After phenol and chloroform extraction and ethanol precipitation, the DNA was digested with Bam HI, electrophoresed on a 6 percent polyacrylamide gel, and the large (4,300 bp) vector fragment recovered by electroelution.

The expression plasmid was assembled by ligating together 0.2 pg of vector, 0.06 pg of the 125 bp blunt end-Nar I fragment and 0.6 µg of the 1645 bp Nar lbgl fragment with 10 units of T₄ DNA ligase for 7 hours at room temperature and used to transform E. coli strain 294 (ATCC No. 31446) to ampicillin resistance. Plasmid DNA was prepared from 26 of the colonies and digested with Xba I and Eco RI. Twelve of these plasmids contained the desired 415 bp Xba IEco RI and 472 bp Eco RI-fragments. DNA sequence analysis verified that several of these plasmids had an ATG initiation codon correctly placed at the start of amino acid number 69 (serine). One of these plasmids, pΔRIPA° was tested and produced the desired tissue plasminogen activator (Figure 7).

### E.1.H. Full length tissue plasminogen activator cDNA

### a). Preparation of a colony library containing N-terminal tissue plasminogen activator sequences

0.4 µg of the synthetic oligonucleotide 5'TTCTGAGCACAGGGCG3' was used for priming 7.5 µg of gel fraction number 8 mRNA (supra) to prepare double stranded cDNA by standard procedures (65, 66). The cDNA was size fractionated on a 6 percent polyacryalmide gel. A size fraction greater than 300 base pairs (36 ng) was electroeluted. 5 ng cDNA was extended with deoxy(C) residues using terminal deoxycytidyl transferase (67) and annealed with 50 ng of the plasmid pBR322 (68) which had been similarly tailed with deoxy(G) residues at the Pst I site (67). The annealed mixture was then transformed into E. coli K12 strain 294. Approximately 1,500 transformants were obtained.

### b). Southern hybridization of human genomic DNA

Since the cDNA priming reaction had been done using a synthetic fragment that hybridized 13 base pairs from the N-terminal of clone pPA25E10, no convenient restriction fragment was available in this 29 base pair region (which includes the 16-mer sequence) for screening the cDNA clones. Therefore, it was necessary to isolate a human tissue plasminogen activator genomic clone in orderto identify any primer extended cDNAclones containing N-terminal tissue plasminogen activator coding sequences. The first step in this process involved establishing the fact that only a single homologous tissue plasminogen activator gene is present in human gemomic DNA. To determine this, a Southern hybridization was performed. In this procedure (77), 5 pg of high molecular weight human lymphocyte DNA (prepared as in 80) was digested to completion with various restriction endonucleases, electrophoresed on 1.0 percent agarose gels (81) and blotted to a nitrocellulose filter (77). A3^{2P}-labelled DNA probe was prepared (76) from the 5' end of the cDNA insert of pPA25E10 (a 230 bp Hpall-Rsa I fragment) and hybridized (82) with the nitrocellulse filter. 35X10⁶ counts per minute of the probe were hybridized for 40 hours and then washed as described (82). Two endonuclease digestion patterns provide only a single hybridizing DNA fragment: Bgl ll (5.7 Kbp) and Pvu ll (4.2 Kbp). Two hybridizing DNA fragments were observed with Hinc ll (5.1 Kbp and 4.3 Kp). Taken together, these data suggest the presence of only a single tissue plasminogen activator gene in the human genome, and that this gene contains at least one intervening sequence.

### c.) Screening of the human λ phage library for tissue plasminogen activator genes

The strategy used to identify λ phage recombinants carrying tissue plasminogen activator genes consisted in detecting nucleotide homology with a radioactive probe prepared from the tissue plasminogen activator cDNA of pPA25E10. One million recombinant λ phage were plated out on DP 50 Sup F at a density of 10,000 pfu/15 cm plate, and nitrocellulose filter replicas were prepared for each plate by the method of Benton and Davis (78). A ³²P-labelled DNA probe was prepared by standard procedures (83) from a 230 base pair Hpa II-Rsa I fragment located 34 base pairs from the 5' end of the plasmid pPA25E10. Each nitrocellulose filter was prehybridized at 42°C for 2 hours in 50 mM sodium phosphate (pH 6.5), 5X SSC (77), .05 mg/ml sonicated salmon sperm DNA, 5X Denhardt's solution (84), 50 percent formamide and then hybridized with 50×10⁶ counts per minute of the labelled probe in the same solution containing 10 percent sodium dextran sulfate (85). After an overnight incubation at 42°C, the filters were washed 4 times at 50° in 0.2X SSC, 0.1 percent SDA for 30 minutes, once in 2x SSC at room temperature and then exposed to Kodak XR-5 X-ray film with Dupont Cronex intensifying screens overnight. A total of 19 clones were obtained which hybridized with the probe. Phage DNA was prepared as previously described (86) from 6 recombinants. λ Clone C was selected for preparation of a Pvu ll fragment for colony screening. 30 µg of DNA was digested with Pvu I for 1 hour at 37°, and electrophoresed on 1.0 percent agarose gels. A 4.2 Kilobase pair fragment previously shown to contain tissue plasminogen activator sequences was electroeluted and purified. A ³²P labelled probe was prepared by standard procedures (83) for colony hybridizations as described infra. d.) Screening of colony library for 5' tissue plasminogen activator sequences

The colonies were transferred from plates and grown on nitrocellulose filters and the DNA from each colony fixed to the filter by the Grunstein-Hogness procedure (69). A ³²P-labelled probe was made by calf-thymus priming (83) a 4.2 kilobase pair Pvu 11 fragment from an isolated tissue plasminogen activator λ genomic clone. Filters containing the 1,500 transformants were hybridized with 112X106 cpm of ³²P-genomic Pvu II fragment. Hybridization was for 16 hours using conditions described by Fritsch et. al (82). Filters were extensively washed and then exposed to Kodak XR-5 X-ray film with Dupont Lightning-Plus intensifying screens for 16-48 hours. Eighteen colonies clearly hybridized with the genomic probe. Plasmid DNA was isolated from each of these colonies and was bound to nitrocellulose filters and hybridized with the ³²P-labelled synthetic oligonucleotide (16-mer) used for the original priming reaction. Of the 18 clones, seven hybridized with the kinased 16-mer. Upon sequence analysis after subcloning fragments into the m13 vector mp⁷ (73), one clone (pPAl7) was shown to contain the correct 5' N terminal region of tissue plasminogen activator, a signal leader sequence and an 84 bp 5' untranslated region. From the two clones pPA25E10 and pPA17 the complete nucleotide sequence Figure 5 and restriction pattern (Figure 4) of a full length tissue plasminogen activator clone were determined.

The native tissue plasminogen activator molecule has the potential to be stabilized by 17 disulfide bridges based on homology with other serine proteases. There are four potential N-glycosylation sites, three located in the "kringle" regions at asn_{117'} asn₁₈₄, asn₂₁₈ and one potential site in the light chain region, at asn₄₄₈. Variations in the structure of the oligosaccharide ligands may be responsible for the different molecular forms (65,000 and 63,000 mol. wt. species).

### E.1.1. Direct expression of full length tissue plasminogen activator cDNA clone in E. coli

A reconstruction of the entire coding sequence was possible employing the common Hhal restriction endonuclease site shared by both partial clones pPA17 and pPA25E10. A 55 pb Sau3Al-Hhal restriction fragment corresponding to amino acids 5-23 was isolated from the plasmid pPAl7. The Sau3AI restriction site was located at codon four of the presumed mature coding sequence and was used to remove the signal peptide coding region. A 263 bp Hhal -Narl fragment (coding for amino acids 24-110) was also isolated from plasmid pPA25E10. Two synthetic deoxyoligonucleotides were designed which restore the codons for amino acids 1-4, incorporate an ATG translational initiation codon and create an EcoRl cohesive terminus. These free fragments were then ligated together to from a 338 bp fragment coding for amino acids 1-110.

This fragment and a 1645 bp Narl-Bg/l fragment from pPA25 E10 were then ligated between the EcoRl and Bg/ II sites of the plasmid pLetFAtrp1 03 (53) to give the expression plasmid pt-PAtrp12. The cloned t-PA gene is transcribed under the control of a 300 bp fragment of the E. coli trp operon which contains the trp promoter, operator, and the Shine-Dalgarno sequence of the trp leader peptide but lacks the leader peptide ATG initiation codon (52).

E. coliK12 strain W3110 (ATCC No. 27325) containing the plasmid pt-PAtrp12 was grown, and extracts prepared for assay of fibrinolytic activity. One method used for measuring tissue plasminogen activator activity is the fibrin plate assay (87). This measures the amount of plasmin formation by measuring the extent of plasmin digestion of fibrin in an agarose plate containing plasminogen and fibrin. Plasmin produces a clear lysis zone in the fibrin plate and the area of this zone can be correlated to the amount of tissue plasminogen activator in the sample. When extracts from pt-PAtrp12 clones are tested for tissue plasminogen activator activity using the fibrin plate assay a clear zone of lysis is evident. This fibrinolytic activity is inhibited by anti t-PA IgG but not by preimmune IgG or anti-urokinase IgG and no activity is seen from an extract prepared from cells containing as a control the leukocyte interferon plasmid pLeIFAtrp103. Using a standard curve of purified t-PA, it is possible to estimate that approximately 20 units of extracted activity per 109 cells are obtained (for purified t-PA, 90_{;}000 Plough units=1 mg) (Figure 10).

### E.1.J Sequence analysis

Sequence analysis was based on the Edman degradation (83b). The sample was introduced into the cup of the Beckman^{*}8908 or 890C spinning cup sequencer. Polybrene^{*} (poly N,N,NlN^{l} -tetramethyl - N-trimethylenehexameth-ylene diammonium diacetate) was used as a carrier in the cup (63C). The sequencer was modified with a cold trap and some program changes to reduce background peaks. The reagents were Beckman's sequence grade 0.1 molar Quadrol^{*} buffer, phenylisothiocyanate, and heptafluorabutyric acid.

The collected Edman cycles were manually converted to 2 - anilino - 5 - thiazolinone derivatives. The 1 - chlorobutane was dried under nitrogen. Then 1.0 N HCI in water was added to the 2 - anilino - 5 - thiazolinone and heated to 70°C for 10 min to convert it into the 3 - phenyl - 2 - thiohydantoin (PTH derivative). The PTH-amino-acid residue was then dissolved in 50 percent acetonitrile and water and injected into a reverse-phase high-pressure liquid chromatograph. Each PTH-amino acid was then identified by comparison to the retention times of a standard mixture of PTH-^{*} Trade Mark amino acids that was introduced into the conversion vial and treated the same way as a cycle from the sequencer.

### E.1.K. Assays for detection of expression of tissue plasminogen activator

### 1. Direct assay of plasmin formation

### a. Theory

A sensitive assay for tissue plasminogen activator can be obtained by monitoring the tissue plasminogen activator catalyzed conversion of plasminogen to plasmin. Plasmin is an enzyme for which there are chromogenic substrate assays. These assays are based on the proteolytic cleavage of a tripeptide from a chromophoric group. The rate of cleavage is directly related to both the specificity and the concentration of the protease being tested. The basis of the assay is the determination of the amount of plasmin formed following incubation of the tissue plasminogen activator containing solution with a solution of plasminogen. The greater the amount of activator, the greater the amount of plasmin formed. Plasmin is measured by monitoring its cleavage of the chromogenic substrate S2251 (purchased from Kabi Group, Inc., Greenwicht, CT).

### b. Procedure

An aliquot of the sample is mixed with 0.10 ml of 0.7 mgs/ml plasminogen (in 0.05M Tris - HCI, pH 7.4, containing, 0.012 M NaCI) and the volume adjusted to 0.15 ml. The mixture is incubated at 37°C for 10 minutes, 0.35 ml of S2251 (1.0 mM solution in above buffer) is added, and the reaction continued for 30 minutes at 37°C. Glacial acetic acid (25 µL) is added to terminate the reaction. The samples are centrifuged and the absorbance at 405 nm is measured. Quantitation of the amount of activity is obtained by comparison with a standard urokinase solution. The assay conditions for detection of the full length tissue plasminogen activator were modified by the addition of fibrinogen (0.2 mg) to the solution. Fibrinogen results in a stimulation of the activity of tissue plasminogen activator observed therefore resulting in somewhat elevated level of activity. Activity was recorded in Plough units, wherein 90,000 Plough units is equal to the activity exhibited by 1 mg of purified tissue plasminogen activator.

### 2. Indirect assay of plasmin formation

### a. Theory

A sensitive assay for tissue plasminogen activator activity has been developed (87). The assay is based on determination of plasmin formation by measuring the extent of plasmin digestion of fibrin in an agar plate containing fibrin and plasminogen. Plasmin produces a clear lysis zone in the fibrin plate. The area of this lysis zone can be correlated to the amount of tissue plasminogen activator in the sample.

### b. Procedure

Following the procedure of Granelli-Piperno and Reich (87), the plates were incubated 3.5 hours at 37°C and lysis zones measured. Quantitation was obtained by comparison to a standard urokinase solution.

### E.1.L. Detection of tissue plasminogen activator activity

### 1. Bacterial growth and sample preparation

A colony of E. colicontaining the plasmid (pΔRIPA°) was inoculated into a test tube containing 5 mL of LB growth media containing 20 pg/ml ampicillin. The cells were grown overnight at 37°C. An aliquot of this culture was diluted 1: 100 into 300 ml of M9 media containing 20 pg/ml ampicillin. The cells were grown in a shaker flask at 37°C for four hours, with a resulting absorbance at 550 nm of 0.419. The tryptophan analog indole acrylic acid was added to a concentrated of 30 pg/ml. The cells were incubated 90 minutes, with a resulting absorbance at 550 nm of 0.628. The cells were harvested by centrifugation and resuspended in 0.8 ml of 0.01 M Tris, pH 8.0, containing 0.01 M EDTA. The resulting suspension was stirred rapidly at room temperature for 18 hours. The sample was centrifuged and the supernatant assayed for tissue plasminogen activator activity.

For expression of pt-PAtrpl2, see the detailed description under the Figure 10 legend.

### 2. Activity detection.

Tables 1 and 2 show the results of the activation of plasminogen by respective E. coli extracts when assayed. An activity is generated which is dependent on the presence of plasminogen (Table 1). This activity is not affected by pre-immune serum of a rabbit but is markedly inhibited by antiserum which was raised against purified melanoma cell derived tissue plasminogen activator (88) (Tables 1 and 2). This demonstrates that the E. coli extracts are producing a plasminogen activating activity which is inhibited by antibodies against the tissue plasminogen activator.

Figure 7 shows the result of a fibrin plate assay for fibrinolytic activity. A standard amount of urokinase was added to the center row in concentrations, from left to right, of 0.24, 0.14_{;} 0.10, 0.05 and 0.02 Plough Units. The bottom row is samples of natural tissue plasminogen activator, with the same amount of enzyme in each well. The wells contain, from left to right, tissue plasminogen activator, anti-plasminogen activator plus preimmune serum, and tissue plasminogen activator plus tissue plasminogen activator antibodies. The wells in the top row each contain 8 µl of the recombinant tissue plasminogen activator E. coli extracts. The first well is the extract alone, the second well has pre-immune serum added, and the third well has the tissue plasminogen activator antibodies added. It is obvious that the preimmune serum does not affect natural or recombinant tissue plasminogen activator, and that tissue plasminogen activator antibodies inhibit the activity of natural as well as the E. coli extracts. Based on the urokinase standards, the extracts contain slightly less than 2.5 Plough units per ml. This compares favorably with the value obtained in Table 1 of 1.3 Plough units per ml.

Tables 1 and 2 set forth the results of assays performed as described above in E.1.K.1 b.:

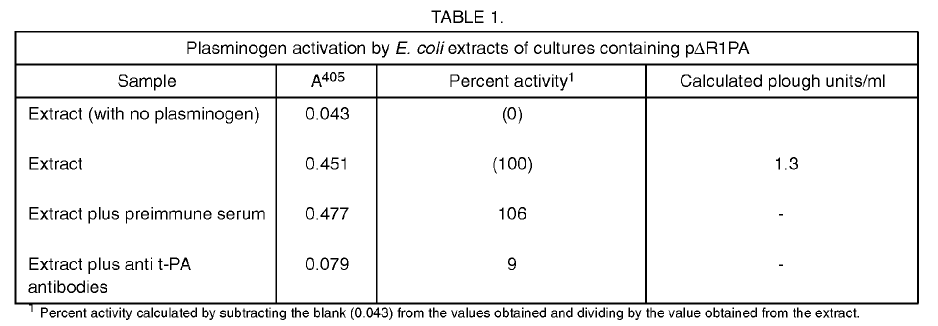

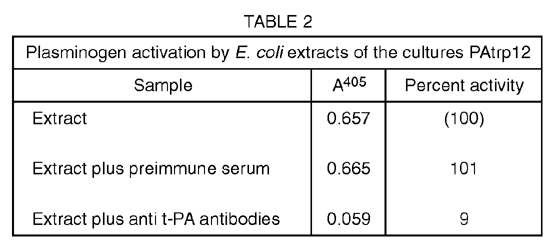

Figure 10 represents the results of a fibrin plate assay performed with extracts from 10 L fermentation cultures of E. coli containing a tissue plasminogen activator expressing plasmid. The fibrinolytic activity of the tissue plasminogen activator containing extract is represented in Figure 10 by Well A. This fibrinolytic activity is inhibited by anti t-PA IgG (Well C) but not by preimmune IgG (Well B) or anti urokinase IgG (Well D) and no activity is seen from an extract prepared from cells containing as a control the leukocyte interferon plasmid pLeIFAtrp103 (Well H).

### E. 2 Production of t-PA using DHFR protein with a low binding affinity for MTX

### E.2.A Vector construction

The sequence encoding human tissue plasminogen activator (t-PA) is inserted into an expression plasmid encoding a mutant DHFR with low binding affinity for MTX.

Three fragments from overlapping t-PA plasmids, pPA25E10, and pPAl7, and pt-PAtrp12 (supra) were prepared as follows: Plasmid pPA17 was digested with Dde I, filled in using Klenow DNA polymerase 1, and subcut with Pst I; the approximately 200 bp fragment containing 5' terminal t-PA sequence thus generated was isolated. The second t-PA fragment was obtained by digesting pt-PAtrp12 with Pst I and Nar I and isolating the approximately 310 bp fragment. The third t-PA fragment was obtained by digesting pPA25E10 with Nar I and Bgl 11 and isolating the approximately 1645 bp fragment which contains, in addition to much of the t-PA coding region, some 3' non-translated sequences.

Plasmid pE342 which expresses HBV surface antigen (also referred to as pHBs348-E) has been described by Levinson et al, in EP 73656. Briefly, the origin of the Simian virus SV40 was isolated by digesting SV40 DNA with Hindlll, and converting the Hindll ends to EcoRl ends by the addition of a converter (AGCTGAATTC). This DNA was cut with Pvull, and RI linkers added. Following digestion with EcoRl, the 348 base-pair fragment spanning the origin was isolated by polyacrylamide gel electrophoresis and electroelution, and cloned in pBR322. Expression plasmid pHBs348-E was constructed by cloning the 1986 base-pair fragment resulting from EcoRl and Bglll digestion of HBV (Animal virus Genetics, (Ch. 5) Acad. Press, N.Y (1980)) (which spans the gene encoding HBsAg) into the plasmid pML (Lusky et al., Nature, 293: 79 (1981) at the EcoRl and BamHl sites. (pML is a derivative of pBR322 which has a deletion eliminating sequences which are inhibitory to plasmid replication in monkey cells). The resulting plasmid (pRl-Bgl) was then linearized with EcoRl, and the 348 base-pair fragment representing the SV40 origin region was introduced into the EcoRl site of pRl-Bgl. The origin fragment can insert in either orientation. Since this fragment encodes both the early and late SV40 promoters in addition to the origin of replication, HBV genes could be expressed under the control of either promoter depending on this orientation (pHBS348-E representing HBs expressed under control of the early promoter). pE342 is modified by partially digesting with Eco RI, filling in the cleaved site using Klenow DNA ploymerase I, and ligating the plasmid back together, thus removing the Eco RI site preceding the SV40 origin in pE342. The resulting plasmid, designated pE342AR1, is digested with Eco RI, filled in using Klenow DNA polymerase I, and subcut with Bam HI. After electrophoresing on acrylamide gel, the approximately 3500 bp fragment is electroeluted, phenolchloroform extracted, and ethanol precipitated as above.

The thus prepared p342E 3500 bp vector, and above described t-PA fragments comprising approximately 2160 bp were ligated together using standard techniques. A plasmid containing the three t-PA encoding fragments in the proper orientation was isolated, characterized, and designated pE342-t-PA. This plasmid was digested with Sac II and treated with bacterial alkaline phosphatase (BRL). To provide the DHFR sequence (along with control sequences for its expression) an approximately 1700 bp fragment was generated by Sacll digestion of pEHER. (pEHER is a plasmid expressing mutant DHFR). This fragment was ligated into the pE342-t-PA plasmid to create pETPAER400, a plasmid which is analogous to pEHER except that the HBsAg coding region has been replaced by the cDNA sequences from t-PA

### E.2.B Expression and amplification of the t-PA sequence

pETPAER400 (pETPER) was transfected into both dhfr CHO-DUX B11 cells and DHFR+ CHO-K1 (ATCC CCL61 ) cells by method of Graham and Van der Eb (supra). Transformed dhfr cells were selected by growth in glycine, hypoxanthine and thymidine deficient medium. Transformed DHFR⁺ cells were selected by growth in ≥100 nM MTX. Colonies which arose on the appropriate selection medium were isolated using cloning rings and propagated in the same medium to several generations.

For amplification cells from the colonies are split into media containing 5_{X}10⁴, 105^{,} 2.5_{X}10⁵, 5_{X}10⁵, and 106 nM MTX and passaged several times. Cells are plated at very low (102⁻103 cells/plate) cell densities in 10 cm dishes and the resulting colonies are isolated.

### E.2.C Assay methods

Expression of t-PA in the transfected amplified colonies may conveniently be assay by the methods similar to those set forth in E.1.K.1.b (supra).

Coamplification of DHFR and t-PA sequences is assayed by isolating DNA from confluent monolayers of amplified colonies as follows: Confluent monolayers in 150 mm plates are washed with 50 ml sterile PBS and lysed by the addition of 5 ml of 0.1 percent SDS, 0.4 M CaCl₂, 0.1 M EDTA, pH 8. After 5-10 minutes, the mixture is removed, phenol extracted, chloroform extracted, and ethanol precipitated. The DNA is resuspended in 1 ml (per 100 mm plate) 10 mM Tris-HCI pH 8, 1 mM EDTA (TE), RNase added to 0.1 mg/ml, and the solution incubated 30 minutes at 37°C. SDS is then added to 0.1 percent and pronase (Sigma) is added to 0.5 mg/ml. After 3-16 hours incubation at 37°C, the solution is again phenol extracted, chloroform extracted, and ethanol precipitated. The DNA pellet is resuspended in 0.5 ml water and digested with restriction enzymes. Approximately 5-10 ₀ pg of digested DNA is electrophoresed in an agarose gel [1 percent agarose in Tris-acetate buffer (40 mM Tris, 1 mM EDTA, made to pH 8.2 with acetic acid)] ; Crouse, et al, J. Biol. Chem., 257: 7887 (1982)). After bromphenol blue dye had migrated 2/3 of the way down the gel, the gel is removed and stained with ethidium bromide. After visualizing the DNA with ultraviolet light, the DNA is transferred from the gel to nitrocellulose filters according to the procedure of Southern (J. Mol. BioL 98: 503 (1975)). The filters are then hybridized with a nick translated probe made from the 1700 bp Sacl I fragment of pEHER (prepared and hybridized as described above), or from the approximately 1970 bp Bgl II fragment of pETPER.

### E.3 Production of t-PA in conjunction with wild type DHFR protein

### E.3.A. Vector construction

In a manner analogous to that used in the construction of pETPER, plasmid pETPFR was conducted containing the DNA sequence encoding wild type DHFR. The construction was as described in Example E.2.A except that in place of plasmid pEHER as a source for the DHFR protein gene sequence, the plasmid pE342 . HBV · E400 . D22 was substituted.

The plasmid pE342 . HBV . E400 . D22 is the same as pEHER except for a single base pair difference between wild type and mutant DHFR. Thus the resulting plasmid pETPFR is analogous in every way to pETPER except that the DNA sequence encoding for wild type DHFR is substituted for that of the mutant.

### E.3.B Expression of t-PA sequence

pETPFR was used to transfect DHFR deficient CHO cells (Urlaub and Chasin (supra)) using the calcium phosphate precipitation method of Graham and Van der Eb. Twenty-one colonies which arose on the selective medium (-HGT) were assayed by detection of plasmin formation as assessed by the digestion of fibrin in an agar plate containing fibrin and plasminogen, described by Granelli-Piperno, et al, J. Exp. Med., 148: 223 (1978).

Four of the most strongly positive clones were then assayed quantitatively for plasmin formation on a per cell basis according to the method set forth in E.1.K.1.b.

Upon such quantitative determination it was found that the four clones, tested exhibited the same or comparable t-PA secretion into the medium, determined as units/cell/day. Subclones were prepared by transferring inocula from two of the clones into separate plates containing -HGT medium. Two of the resulting subclones, 188 and 1 were used for further analysis.

### E.3.C Amplification and t-PA production levels

The above subclones were plated at 2X10⁵ cells per 100 mm plates in 50 nM MTX to promote amplification. Those cells which survived, when assayed as described above, gave, in all cases, about 10 times the unamplified amount of tissue plasminogen activator activity. Two of these clones were chosen for further study and were named 1-15 and 18B-9.

Subclone 1-15 was further amplified by seeding 2X10⁵ cells in 100 mm plates containing 500 nM MTX. Assay of the cells thus amplified yielded a further increase (of about 3 fold) in t-PA production; when assayed quantitatively by the method of C.1.C, levels were in the range of 7_{X}10-4 units/cell/day. A portion of these amplified cells was then transferred and maintained in the presence of 10,000 nM MTX. Subclones of 1-15, and 18B-9 were further tested after being maintained for approximately 1-2 months at the conditions specified in Table 3.

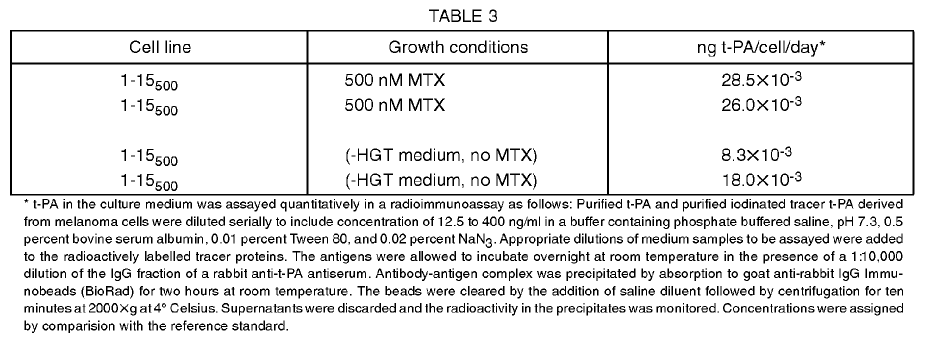

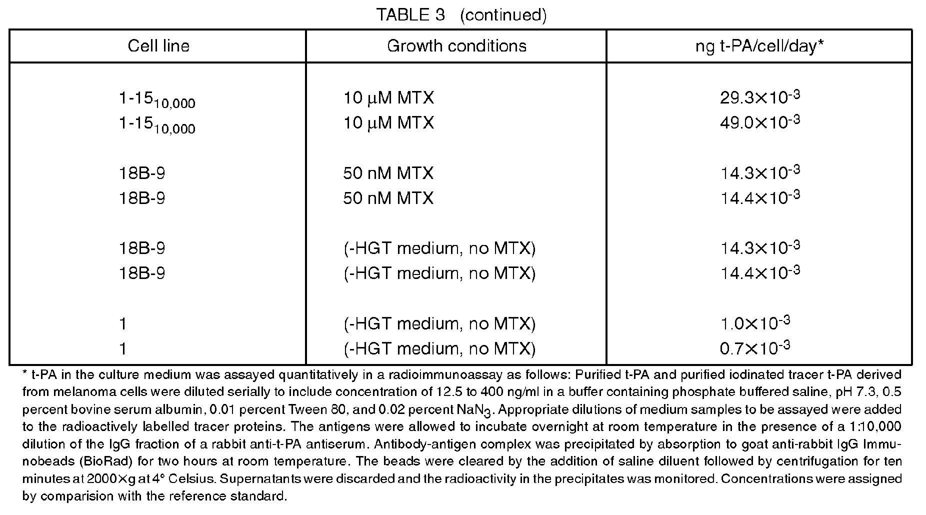

The cell lines are as follows: Cell line "1" is an unamplified clone from the original set of four. "1-15₅₀₀" is an amplified subclone of cell line "1 " which was amplified initially in 50 nM MTX to give 1-15 and then transferred for further amplification into 500 nM MTX. 1-15_{10,000} is subclone of 1-15₅₀ₒ which has been further amplified in the presence of 10_{;}000 nM MTX. Cell line 18B-9 is a subclone of one of the original four detected which had been amplified on 50 nM MTX.

All of the amplified cells show increased levels of t-PA production over that exhibited by the unamplified cell culture. Even the unamplified culture produces amounts of t-Pa greater than 0.5 pg/cell/day, amplification results in levels approaching 50 pg/cell/day.

### F. Pharmaceutical compositions

The compounds of the present invention can be formulated according to known methods to prepare pharmaceutically useful compositions, whereby the human tissue plasminogen activator product hereof is combined in admixture with a pharmaceutically acceptable carrier vehicle. Suitable vehicles and their formulation, inclusive of other human proteins, e.g. human serum albumin, are described for example in Remington's Pharmaceutical Sciences by E. W. Martin, which is hereby incorporated by reference. Such compositions will contain an effective amount of the protein hereof together with a suitable amount of vehicle in order to prepare pharmaceutically acceptable compositions suitable for effective administration to the host.

For example, the human tissue plasminogen activator hereof may be parenterally administered to subjects suffering from cardiovascular diseases or conditions. Dosage and dose rate may parallel that currently in use in clinical investigations of other cardiovascular, thrombolytic agents, e.g., about 440 IU/kg. body weight as an intravenous priming dose followed by a continuous intravenous infusion at about 440 IU/kg./hr. for 12 hours, in patients suffering from pulmonary embolism.

As one example of an appropriate dosage form for essentially homogeneous human tissue plasminogen activator in parenteral form applicable herein, a vial containing 25000 IU tissue plasminogen activator activity, 25 mg. mannitol and 45 mg. NaCl, may be reconstituted with 5 ml. sterile water for injection and mixed with a suitable volume of 0.9 percent Sodium Chloride Injection or 5 percent Dextrose Injection for intravenous administration.

### G. Detailed description of recombinant human t-PA

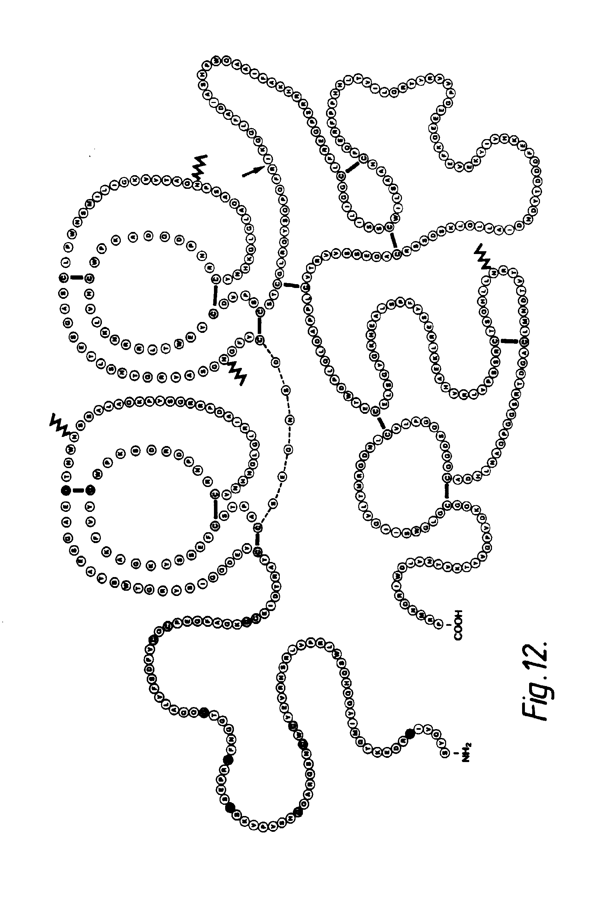

The structure of the particular embodiment of human t-PA prepared in the examples herein has been studied in some detail, both by elucidation of the gene coding sequence and by protein biochemistry techniques. The current understanding of the protein structure is illustrated in Figure 12.

It has also previously been demonstrated by Collen and coworkers (88) that two chain human t-PA is formed by proteolytic cleavage of the single chain molecule into two polypeptides connected by disulfide bonding. The present work permits the conclusion that the heavy chain (30882 mol. wt.) is derived from the NH2 terminal part and the light chain (28126 mol. wt.) comprises the COOH-terminal region. N-terminal sequencing of the two chain molecule suggests that the two chain form is generated by cleavage of a single arginyl-isoleucine bond (Figure 12; arrow depicted).

The primary structure of a portion of the heavy chain region of human t-PA (Figure 12) reveals a high degree of sequence homology with the "kringle" regions of plasminogen (89) and prothrombin (40, 41 ). "Kringle" region refers to a characteristic triple disulfide structure originally discovered in the "pro"-fragment of prothrombin, first described in detail by Magnusson et al. (91, 92). From the primary sequence of t-PA, two so-called "kringle" regions, of 82 amino acids each, that share a high degree of homology with the 5 "kringle" regions of plasminogen are apparent. The remaining N-terminal 91 amino acids share little homology to the conventional "kringle" region. One can speculate however that this region may also assume a structure containing multiple disulfide bonds as 11 additional cysteine residues are found here.

The catalytic site of the light chain of human t-PA, the so-called serine protease region, as in other serine enzymes, is most likely formed by the histidine₃₂₂, aspartic₃₇₁ and serine₄₇₈ residues. Furthermore, the amino acid sequences surrounding these residues are very homologous to corresponding parts of other serine proteases such as trypsin, prothrombin and plasminogen.

Notwithstanding that reference has been made to particular preferred embodiments, it will be understood that the present invention is not to be construed as limited to such, rather to the lawful scope of the appended claims.

### Bibliography

1. United States Patent No. 3355361.
2. United States Patent No. 3926727.
3. United States Patent No. 4029767.
4. United States Patent No. 4258030.
5. United States Patent No. 4271150.
6. European Patent Application Publn. No. 0037687.
7. Rijken, D.C., "Plasminogen Activator from Human Tissue," Krips Repro Meppel, 1980
8. United States Patent No. 3555000.
9. United States Patent No. 3998947.
10. United States Patent No. 4245051.
11 . European Patent Application Publn. No. 0023860.
12. United States Patent No. 4083961.
13. United States Patent No. 4177262.
14. United States Patent No. 4082612.
15. Wallen, P., Proc. Serono Symp. 9,91 (1977).
16. Thorsen, S., et aL, Thrombos. Diathes. haemorrh. 28, 65 (1972).
17. Collen, Thrombos. Haemostas. 43, 77 (1980).
18. Wiman et al., Nature 272, 549 (1978).
19. European Patent Application Publn. No. 0041766.
20. Weimar, W., et al., The Lancet Vol. II, No. 8254, p. 1018 (1981).
21. British Patent Application Publn. No. 2007676A.
22. Wetzel, American Scientist 68, 664 (1980).
23. Microbiology, 2d Ed., Harper and Row Publications, Inc., Hagerstown, Maryland (1973), esp. pp. 1122 et seq.
24. Scientific American 245, 106 (1981).
25. British Patent Application Publn. No. 2055382A.
26. German Offenlegungsschrift 2644432.
27. Chang et al., Nature 275, 617 (1978).
28. Itakura etal., Science 198, 1056 (1977).
29. Goeddel et aL, Nucleic Acids Research 8, 4057 (1980).
30. European Patent Application Publn. No. 0036776.
31. Siebenlist et al., Cell 20,269 (1980).
32. Stinchcomb et al., Nature 282, 39 (1979).
33. Kingsman et aL, Gene 7, 141 (1979).
34. Tschumper et al., Gene 10, 157 (1980).
35. Mortimer et al, Microbiological Reviews 44, 519 (1980).
36. Miozzari et aL, Journal of Bacteriology 134, 48 (1978).
37. Jones, Genetics 85, 23 (1977).
38. Hitzeman, et al., J. Biol. Chem. 255, 12073 (1980).
39. Hess et al., J. Adv Enzyme Regul. 7, 149 (1968).
40. Holland et al., Biochemistry 17, 4900 (1978).
41. Bostian et al,. Proc. Natl. Acad. Sci. (USA) 77, 4504 (1980).
42. The Molecular Biology of Yeast (Aug 11-18, 1981), Cold Spring Harbor Laboratory, Cold Spring Harbor, New York.
43. Chambon, Ann. Rev. Biochemistry, 44, 613 (1975).
44. Tissue Culture, Academic Press, Kruse and Patterson eds, (1973).
45. Gluzman, Cell23, 175 (1981).
46. Bolivar et al., Gene 2, 95 (1977).
47. Lusky et al., Nature 293, 79 (1981).
48. Gluzman et al., Cold Spring Harbor Symp. Quant. Biol. 44, 293 (1980).
49. Fiers et al., Nature 273, 113 (1978).
50. Reddy et al., Science 200, 494 (1978).
51. Crea et al, Nucleic Acids Research 8, 2331 (1980).
52. Goeddel et al., Nature 287, 411 (1980).
53. Gray et al, Nature 295, 503 (1982).
54. Oppermann et al., Virology 108, 47 (1981).
55. Ward etal., J. Virol. 9, 61 (1972).
56. Aviv et al., Proc. Natl. Acad. Sci. (USA) 69, 1408 (1972).
57. Lehrach et al., Biochemistry 16, 4743 (1977).
58. Lynch et al., Virology 98, 251 (1979).
59. Lodish Ann. Rev. of Biochem. 45, 40 (1976).
60. Pelham et al., Eur. J. Biochem. 43, 247 (1974).
61. Blobel, etal., J. Cell Biology 67, 852 (1975).
62. Shields et al., J. Biol. Chemistry253, 3753 (1978).
63. Laemmli, Nature 227, 680 (1970).
64. Bonner et al., Eur. J. Biochem. 46, 83 (1974).
65. Goeddel et al., Nature 281, 544 (1979).
66. Wickens et al., J. Biol. Chem. 253, 2483 (1978).
67. Chang et al., Nature 275, 617 (1978).
68. Bolivar et al., Gene 2, 95 (1977).
69. Grunstein et al., Proc. Natl. Acad. Sci. U.S.A. 72, 3961 (1975).
70. Hanahan et al, Gene 10, 63 (1980).
71. Birnboim et al., Nucleic Acids Res. 7, 1513 (1979).
72. Smith, Methods Enzymol. 65, 499 (1980).
73. Messing et al., Nucleic Acids Res. 9, 309 (1981).
74. Maxam et al., Methods in EnzymoL 65, 499 (1980).
75. Crea et al, Proc. Natl. Acad. Sci. 75, 5765 (1978).
76. Lawn et al., Cell 15, 1157 (1978).
77. Southern, J. Mol. Biol. 98, 503 (1975).
78. Benton et al., Science 196, 180 (1977).
79. McGrath and Levinson, Nature 295, 423 (1982).
80. Blin et al., Nucleic Acid Res. 3, 2303 (1976).
81. Lawn et al., Science 212, 1159 (1981).
82. Fritsch et al., Cell 19, 959 (1980).
83. Taylor et al., Biochim, Biophys. Acta 442, 324 (1976).
83b. Edman et al., European J. Biochem. 1, 80 (1967).
84. Denhardt, Biochem. Biophys. Res. Comm. 23, 641 (1966).
85. Wahl et al., Proc. Natl. Acad. Sci. (USA) 76, 3683 (1979).
86. Davis et al., Advanced Bacterial Genetics, Cold Spring Harbor Laboratory, New York (1980).
87. Granelli-Piperno et al., J. Exp. Med. 148, 223 (1978).
88. Rijken et al., J. Biol. Chem. 256, 7035 (1981).
89. Sottrup-Jensen et al, Progress in Chemical Fibrinolysis and Thrombolysis, Vol. 3, Raven Press, N. Y. p. 191 (1978).
90. Sothrup-Jensen etal., Proc. Natl. Acad. Sci. (USA) 72, 2577 (1975).
91. Magnussen et al., Proteolysis and Physiological Regulation, Ribbons et al., Eds., Academic Press, New York,
p. 203 (1976). Laboratory, N. Y, p. 123
92. Magnussen et al., Proteases and Biological Control, Cold Spring Harbor Laboratory, N. Y, p. 123 (1975).
93. Miller, Experiments in Molecular Genetics, p.431-3, Cold Spring Harbor Lab., Cold Spring Harbor, New York (1972).
94. Reich, E., Proteases and Biological Control, (Ibid) p. 333-341.
95. Matsuo, O., et al., Throm. Haemostasis 45225 (1981).
96. Koringer, C., et al., Throm. Haemostasis 46 561, 662 (1981).
97. Hoylaerts, M. et aL, J. Biol. Chem. 2572912 (1982).
98. Koringer, C., et al. Thromb. Haemostasis 46 685 (1981).

## Claims (Claims for the following Contracting State(s) : BE, CH, DE, FR, GB, IT, LI, LU, NL, SE)

1. A process which comprises the preparation of a protein which has human tissue plasminogen activator function, in particular, it is capable of catalyzing the conversion of plasminogen to plasmin, it binds to fibrin, and is classified as a t-PA based on immunological properties, by expression in a recombinant host organism of transforming DNA encoding the protein; wherein said protein comprises a sequence of 527 amino acids from N-terminal serine to C-terminal proline encodable by cDNA derived from mRNA extracted from the Bowes melanoma cell line, and which
(a) has the restriction pattern shown in Fig 4 hereof for the putative mature tissue plasminogen activator sequence, and
(b) hybridises strongly with the sequences:
(i) 5'-TCACAGTACTCCCA-3'
(ii) 5'-TTCTGAGCACAGGGCG-3'
(iii) a 4.2kb Pvu II fragment of human genomic DNA which hybridises strongly with the sequence

30                                                    40
CGG GTG GAA TAT TGC TGG TGC AAC AGT GGC AGG GCA CAG TGC CAC TCA
                    50                                                    60
GTG CCT GTC AAA AGT TGC AGC GAG CCA AGG TGT TTC AAC GGG GGC ACC
                    66
TGC CAG CAG GCC CTG T.

2. A process which comprises the preparation of a protein which has human tissue plasminogen activator function, in particular, it is capable of catalyzing the conversion of plasminogen to plasmin, it binds to fibrin, and is classified as a t-PA based on immunological properties, by expression in a recombinant host organism of transforming DNA encoding the protein, wherein the protein comprises an allele or derivative, by way of amino acid deletion, substitution, insertion, addition or replacement, of said 527 amino acid sequence as defined in claim 1.

3. A process according to claim 1 or claim 2 wherein there is produced a polypeptide which is unaccompanied by glycosylation native to human tissue plasminogen activator.

4. A process according to claim 1 or claim 2 wherein the host organism is an E coli strain.

5. A process according to claim 1 or claim 2 wherein the host organism is mammalian.

6. A process according to claim 5 wherein the host organism is a Chinese hamster ovary cell line.

7. A process according to any one of claims 1 to 6 which includes recovering and purifying the protein.

8. A process which, following the preparation of a protein having tissue plasminogen activator function by a process of any one of claims 1 to 7, comprises the use of said protein in the manufacture of a pharmaceutical preparation.

9. A DNA isolate encoding a said protein as prepared by the process of claim 1 or claim 2.

10. A recombinant cloning vector containing DNA encoding a said protein as prepared by the process of claim 1 or claim 2.

11. A recombinant expression vector which contains DNA encoding a said protein as prepared by the process of claim 1 or claim 2, and which is capable, in a transformant microorganism or cell culture, of expressing the coding sequence to produce the corresponding polypeptide.

12. A microorganism or cell culture transfected with DNA encoding a said protein as prepared by the process of claim 1 or claim 2 and capable of its expression to produce said protein.

13. A microorganism according to claim 12 which is an E coli strain.

14. A cell culture according to claim 12 which is a mammalian cell line.

15. A cell culture according to claim 14 wherein the cell line is a Chinese hamster ovary cell line.

## Claims (Claims for the following Contracting State(s) : AT)

1. A process which comprises the preparation of a protein which has human tissue plasminogen activator function, in particular, it is capable of catalyzing the conversion of plasminogen to plasmin, binds to fibrin, and is classified as a t-PA based on immunological properties, by expression in a recombinant host organism of transforming DNA encoding the protein; wherein said protein comprises a sequence of 527 amino acids from N-terminal serine to C-terminal proline encodable by cDNA derived from mRNA extracted from the Bowes melanoma cell line, and which
(a) has the restriction pattern shown in Fig 4 hereof for the putative mature tissue plasminogen activator sequence, and
(b) hybridises strongly with the sequences:
(i) 5'-TCACAGTACTCCCA-3'
(ii) 5'-TTCTGAGCACAGGGCG-3'
(iii) a 4.2kb Pvu II fragment of human genomic DNA which hybridises strongly with the sequence

30                                    40
CGG GTG GAA TAT TGC TGG TGC AAC AGT GGC AGG GCA CAG TGC CAC TCA
                50                                          60
GTG CCT GTC AAA AGT TGC AGC GAG CCA AGG TGT TTC AAC GGG GGC ACC
                66
TGC CAG CAG GCC CTG T.

2. A process which comprises the preparation of a protein which has human tissue plasminogen activator function, in particular, it is capable of catalyzing the conversion of plasminogen to plasmin, binds to fibrin, and is classified as a t-PA based on immunological properties, by expression in a recombinant host organism of transforming DNA encoding the protein, wherein the protein comprises an allele or derivative, by way of amino acid deletion, substitution, insertion, addition or replacement, of said 527 amino acid sequence as defined in claim 1.

3. A process according to claim 1 or claim 2 wherein there is produced a polypeptide which is unaccompanied by glycosylation native to human tissue plasminogen activator.

4. A process according to claim 1 or claim 2 wherein the host organism is an E coli strain.

5. A process according to claim 1 or claim 2 wherein the host organism is mammalian.

6. A process according to claim 5 wherein the host organism is a Chinese hamster ovary cell line.

7. A process according to any one of claims 1 to 6 which includes recovering and purifying the protein.

8. A process which, following the preparation of a protein having tissue plasminogen activator function by a process of any one of claims 1 to 7, comprises the use of said protein in the manufacture of a pharmaceutical preparation.

9. A process which comprises the preparation of a DNA isolate encoding a said protein as prepared by the process of claim 1 or claim 2.

10. A process which comprises the preparation of a recombinant cloning vector containing DNA encoding a said protein as prepared by the process of claim 1 or claim 2.

11. A process which comprises the preparation of a recombinant expression vector which contains DNA encoding a said protein as prepared by the process of claim 1 or claim 2, and which is capable, in atransformant microorganism or cell culture, of expressing the coding sequence to produce the corresponding polypeptide.

12. A microorganism or cell culture transferred with DNA encoding a said protein as prepared by the process of claim 1 or claim 2 and capable of its expression to produce said protein.

13. A microorganism according to claim 12 which is an E coli strain.

14. A cell culture according to claim 12 which is a mammalian cell line.

15. A cell culture according to claim 14 wherein the cell line is a Chinese hamster ovary cell line.

## Patentansprüche (Patentansprüche für folgende(n) Vertragsstaat(en) : BE, CH, DE, FR, IT, LI, LU, NL, SE)

1. Verfahren, welches umfaßt die Herstellung eines Proteins, das Human-Gewebeplasminogenaktivator-Funktion aufweist, insbesondere imstande ist, die Umwandlung von Plasminogen zu Plasmin zu katalysieren, an Fibrin bindet und auf der Basis immunologischer Eigenschaften als ein t-PA klassifiziert ist, durch Expression von transformierender, für das Protein kodierender DNA in einem rekombinanten Wirtsorganismus; worin das Protein eine Sequenz von 527 Aminosäuren von N-terminalem Serin bis C-terminalem Prolin umfaßt, welche kodierbar ist durch cDNA, die von aus der Bowes-Melanomzellinie extrahierter mRNA abgeleitet ist, und die
(a) das in Figur 4 dargestellte Restriktionsmuster für die mutmaßliche reife Gewebeplasminogenaktivator-Sequenz aufweist, und
(b) stark hybridisiert mit den Sequenzen:
(i) 5'-TCACAGTACTCCCA-3'
(ü) 5'-TTCTGAGCACAGGGCG-3'
(iii) einem 4,2kb PVUII-Fragment genomischer Human-DNA, welches stark hybridisiert mit der Sequenz

30
CGG GTG GAA TAT TGC TGG TGC AAC AGT GGC AGG GCA CAG TGC CAC TCA
50 60
GTG CCT GTC AAA AGT TGC AGC GAG CCA AGG TGT TTC AAC GGG GGC ACC
66
TGC CAG CAG GCC CTG T.

2. Verfahren, welches umfaßt die Herstellung eines Proteins, das Human-Gewebeplasminogenaktivator-Funktion aufweist, insbesondere imstande ist, die Umwandlung von Plasminogen zu Plasmin zu katalysieren, an Fibrin bindet und auf der Basis immunologischer Eigenschaften als ein t-PA klassifiziert ist, durch Expression von transformierender, für das Protein kodierender DNA in einem rekombinanten Wirtsorganismus, worin das Protein ein Allel oder Derivat, infolge Aminosäure-Deletion, -Substitution, -Insertion, -Addition oder -Austausch, der in Anspruch 1 definierten 527-Aminosäuren-Sequenz umfaßt.

3. Verfahren nach Anspruch 1 oder Anspruch 2, worin ein Polypeptid hergestellt wird, das nicht von für Human-Gewebeplasminogenaktivator nativer Glykosylierung begleitet ist.

4. Verfahren nach Anspruch 1 oder Anspruch 2, worin der Wirtsorganismus ein E. coli-Stamm ist.

5. Verfahren nach Anspruch 1 oder Anspruch 2, worin der Wirtsorganismus ein Säuger ist.

6. Verfahren nach Anspruch 5, worin der Wirtsorganismus eine Ovarial-Zellinie des Chinesischen Hamsters ist.

7. Verfahren nach irgendeinem der Ansprüche 1 bis 6, welches die Gewinnung und Reinigung des Proteins einschließt.

8. Verfahren, welches nach der Herstellung eines Proteins mit Gewebeplasminogenaktivator-Funktion nach einem Verfahren nach irgendeinem der Ansprüche 1 bis 7 die Verwendung des Proteins bei der Herstellung eines pharmazeutischen Präparats umfaßt.

9. DNA-Isolat, kodierend für ein Protein wie nach dem Verfahren von Anspruch 1 oder Anspruch 2 hergestellt.

10. Rekombinanter Klonierungsvektor, der DNA enthält, die für ein Protein, wie nach dem Verfahren von Anspruch 1 oder Anspruch 2 hergestellt, kodiert.

11. Rekombinanter Expressionsvektor, welcher DNA enthält, die für ein Protein, wie nach dem Verfahren von Anspruch 1 oder Anspruch 2 hergestellt, kodiert, und der imstande ist, in einem transformanten Mikroorganismus oder einer transformanten Zellkultur die kodierende Sequenz zu exprimieren, um das entsprechende Polypeptid herzustellen.

12. Mikroorganismus oder Zellkultur, transfiziert mit DNA, die für ein Protein, wie nach dem Verfahren von Anspruch 1 oder Anspruch 2 hergestellt, kodiert, und der oder die zu deren Expression imstande ist, um das Protein herzustellen.

13. Mikroorganismus nach Anspruch 12_{;} der ein E. coli-Stamm ist

14. Zellkultur nach Anspruch 12, die eine Säuger-Zellinie ist.

15. Zellkultur nach Anspruch 14, worin die Zellinie eine Ovarial-Zellinie des Chinesischen Hamsters ist.

## Patentansprüche (Patentansprüche für folgende(n) Vertragsstaat(en) : AT)

1. Verfahren, welches umfaßt die Herstellung eines Proteins, das Human-Gewebeplasminogenaktivator-Funktion aufweist, insbesondere imstande ist, die Umwandlung von Plasminogen zu Plasmin zu katalysieren, an Fibrin bindet und auf der Basis immunologischer Eigenschaften als ein t-PA klassifiziert ist, durch Expression von transformierender, für das Protein kodierender DNA in einem rekombinanten Wirtsorganismus; worin das Protein eine Sequenz von 527 Aminosäuren von N-terminalem Serin bis C-terminalem Prolin umfaßt, welche kodierbar ist durch cDNA, die von aus der Bowes-Melanomzellinie extrahierter mRNA abgeleitet ist, und die
(a) das in Figur 4 dargestellte Restriktionsmuster für die mutmaßliche reife Gewebeplasminogenaktivator-Sequenz aufweist, und
(b) stark hybridisiert mit den Sequenzen:
(i) 5'-TCACAGTACTCCCA-3'
(ü) 5'-TTCTGAGCACAGGGCG-3'
(iii) einem 4,2kb Pvull-Fragment genomischer Human-DNA, welches stark hybridisiert mit der Sequenz

30                                          40
CGG GTG GAA TAT TGC TGG TGC AAC AGT GGC AGG GCA CAG TGC CAC TCA
                    50                                          60
GTG CCT GTC AAA AGT TGC AGC GAG CCA AGG TGT TTC AAC GGG GGC ACC
                    66
TGC CAG CAG GCC CTG T.

2. Verfahren, welches umfaßt die Herstellung eines Proteins, das Human-Gewebeplasminogenaktivator-Funktion aufweist, insbesondere imstande ist, die Umwandlung von Plasminogen zu Plasmin zu katalysieren, an Fibrin bindet und auf der Basis immunologischer Eigenschaften als ein t-PA klassifiziert ist, durch Expression von transformierender, für das Protein kodierender DNA in einem rekombinanten Wirtsorganismus, worin das Protein ein Allel oder Derivat, infolge Aminosäure-Deletion, -Substitution, -Insertion, -Addition oder -Austausch, der in Anspruch 1 definierten 527-Aminosäuren-Sequenz umfaßt.

3. Verfahren nach Anspruch 1 oder Anspruch 2, worin ein Polypeptid hergestellt wird, das nicht von für Human-Gewebeplasminogenaktivator nativer Glykosylierung be-gleitet ist.

4. Verfahren nach Anspruch 1 oder Anspruch 2, worin der Wirtsorganismus ein E. coli-Stamm ist.

5. Verfahren nach Anspruch 1 oder Anspruch 2, worin der Wirtsorganismus ein Säuger ist.

6. Verfahren nach Anspruch 5, worin der Wirtsorganismus eine Ovarial-Zellinie des Chinesischen Hamsters ist

7. Verfahren nach irgendeinem der Ansprüche 1 bis 6, welches die Gewinnung und Reinigung des Proteins einschließt.

8. Verfahren, welches nach der Herstellung eines Proteins mit Gewebeplasminogenaktivator-Funktion nach einem Verfahren nach irgendeinem der Ansprüche 1 bis 7 die Verwendung des Proteins bei der Herstellung eines pharmazeutischen Präparats umfaßt.

9. Verfahren, welches umfaßt die Herstellung eines DNA-Isolats, das für ein Protein, wie nach dem Verfahren von Anspruch 1 oder Anspruch 2 hergestellt, kodiert.

10. Verfahren, welches umfaßt die Herstellung eines rekombinanten Klonierungsvektors, der DNA enthält, die für ein Protein, wie nach dem Verfahren von Anspruch 1 oder Anspruch 2 hergestellt, kodiert.

11. Verfahren, welches umfaßt die Herstellung eines rekombinanten Expressionsvektors, der DNA enthält, die für ein Protein, wie nach dem Verfahren von Anspruch 1 oder Anspruch 2 hergestellt, kodiert, und der imstande ist, in einem transformanten Mikroorganismus oder einer transformanten Zellkultur die kodierende Sequenz zu exprimieren, um das entsprechende Polypeptid herzustellen.

12. Mikroorganismus oder Zellkultur, transfiziert mit DNA, die für ein Protein, wie nach dem Verfahren von Anspruch 1 oder Anspruch 2 hergestellt, kodiert, und der oder die zu deren Expression imstande ist, um das Protein herzustellen.

13. Mikroorganismus nach Anspruch 12_{;} der ein E. coli-Stamm ist.

14. Zellkultur nach Anspruch 12, die eine Säuger-Zellinie ist.

15. Zellkultur nach Anspruch 14, worin die Zellinie eine Ovarial-Zellinie des Chinesischen Hamsters ist.

## Revendications (Revendications pour l'(les) Etat(s) contractant(s) suivant(s) : BE, CH, DE, FR, GB, IT, LI, LU, NL, SE)

1. Procédé qui comprend la préparation d'une protéine qui présente la fonction de l'activateur du plasminogène tissulaire humain, en particulier qui est capable de catalyser la transformation du plasminogène en plasmine, qui se lie à la fibrine et qui est classée en tant que t-PA sur la base de propriétés immunologiques, par expression dans un organisme hôte recombinant de l'ADN transformant codant pour la protéine ; dans lequel ladite protéine comprend une séquence de 527 aminoacides à partir de la sérine N-terminale jusqu'à la proline C-terminale pouvant être codée par un ADNc dérivé de l'ARNm extrait de la lignée de cellules de mélanome de Bowes, et qui
(a) comporte le schéma de restriction représenté sur la figure 4 de la présente invention pour la séquence putative d'activateur du plasminogène tissulaire mature, et
(b) s'hybride fortement avec les séquences :
(i) 5'-TCACAGTACTCCCA-3',
(ii) 5'-TTCTGAGCACAGGGCG-3'
(iii) un fragment Pvu Il de 4,2kb d'ADN génomique humain qui s'hybride fortement avec la séquence

30                                      40
CGG GTG GAA TAT TGC TGG TGC AAC AGT GGC AGG GCA CAG TGC CAC TCA
                    50                                      60
GTG CCT GTC AAA AGT TGC AGC GAG CCA AGG TGT TTC AAC GGG GGC ACC
                    66
TGC CAG CAG GCC CTG T.

2. Procédé qui comprend la préparation d'une protéine qui présente la fonction de l'activateur du plasminogène tissulaire humain, en particulier qui est capable de catalyser la transformation du plasminogène en plasmine, qui se lie à la fibrine et qui est classée en tant que t-PA sur la base de propriétés immunologiques, par expression dans un organisme hôte recombinant de l'ADN transformant codant pour la protéine, dans lequel la protéine comprend un allèle ou dérivé, par délétion, substitution, insertion, addition ou remplacement d'aminoacides, de ladite séquence de 527 aminoacides répondant à la définition suivant la revendication 1.

3. Procédé suivant la revendication 1 ou la revendication 2, dans lequel est produit un polypeptide qui est non accompagné par la glycosylation naturelle de l'activateur du plasminogène tissulaire humain.

4. Procédé suivant la revendication 1 ou la revendication 2, dans lequel l'organisme hôte est une souche de E. coli.

5. Procédé suivant la revendication 1 ou la revendication 2, dans lequel l'organisme hôte est issu d'un mammifère.

6. Procédé suivant la revendication 5, dans lequel l'organisme hôte est une lignée de cellules d'ovaires de hamster chinois.

7. Procédé suivant l'une quelconque des revendications 1 à 6, qui comprend les étapes consistant à recueillir et purifier la protéine.

8. Procédé qui, après la préparation d'une protéine ayant la fonction de l'activateur du plasminogène tissulaire par un procédé suivant l'une quelconque des revendications 1 à 7, comprend l'utilisation de ladite protéine dans la production d'une préparation pharmaceutique.

9. Isolat d'ADN codant pour une telle protéine préparée par le procédé suivant la revendication 1 ou la revendication 2.

10. Vecteur de clonage recombinant contenant l'ADN codant pour une telle protéine préparée par le procédé suivant la revendication 1 ou la revendication 2.

11. Vecteur d'expression recombinant qui contient l'ADN codant pour une telle protéine préparée par le procédé suivant la revendication 1 ou la revendication 2, et qui est capable, dans une culture de micro-organismes ou cellules transformants, d'exprimer la séquence codante pour la production du polypeptide correspondant.

12. Culture de micro-organismes ou de cellules transfectée avec l'ADN codant pour une telle protéine préparée par le procédé suivant la revendication 1 ou la revendication 2, et capable d'exprimer cet ADN pour produire ladite protéine.

13. Micro-organisme suivant la revendication 12, qui est une souche de E. coli.

14. Culture cellulaire suivant la revendication 12, qui est une lignée cellulaire de mammifère.

15. Culture cellulaire suivant la revendication 14, dans laquelle la lignée cellulaire est une lignée de cellules d'ovaires de hamster chinois.

## Revendications (Revendications pour l'(les) Etat(s) contractant(s) suivant(s) : AT)

1. Procédé qui comprend la préparation d'une protéine qui présente la fonction de l'activateur du plasminogène tissulaire humain, en particulier qui est capable de catalyser la transformation du plasminogène en plasmine, qui se lie à la fibrine et qui est classée en tant que t-PA sur la base de propriétés immunologiques, par expression dans un organisme hôte recombinant de l'ADN transformant codant pour la protéine ; dans lequel ladite protéine comprend une séquence de 527 aminoacides à partir de la sérine N-terminale jusqu'à la proline C-terminale pouvant être codée par un ADNc dérivé de l'ARNm extrait de la lignée de cellules de mélanome de Bowes, et qui
(a) comporte le schéma de restriction représenté sur la figure 4 de la présente invention pour la séquence putative d'activateur du plasminogène tissulaire mature, et
(b) s'hybride fortement avec les séquences :
(i) 5'-TCACAGTACTCCCA-3',
(ii) 5'-TTCTGAGCACAGGGCG-3'
(iii) un fragment Pvu Il de 4,2kb d'ADN génomique humain qui s'hybride fortement avec la séquence

30                                    40
CGG GTG GAA TAT TGC TGG TGC AAC AGT GGC AGG GCA CAG TGC CAC TCA
                50                                          60
GTG CCT GTC AAA AGT TGC AGC GAG CCA AGG TGT TTC AAC GGG GGC ACC
                66
TGC CAG CAG GCC CTG T.

2. Procédé qui comprend la préparation d'une protéine qui présente la fonction de l'activateur du plasminogène tissulaire humain, en particulier qui est capable de catalyser la transformation du plasminogène en plasmine, qui se lie à la fibrine et qui est classée en tant que t-PA sur la base de propriétés immunologiques, par expression dans un organisme hôte recombinant de l'ADN transformant codant pour la protéine, dans lequel la protéine comprend un allèle ou dérivé, par délétion, substitution, insertion, addition ou remplacement d'aminoacides, de la séquence de 527 aminoacides répondant à la définition suivant la revendication 1.

3. Procédé suivant la revendication 1 ou la revendication 2, dans lequel est produit un polypeptide qui est non accompagné par la glycosylation naturelle de l'activateur du plasminogène tissulaire humain.

4. Procédé suivant la revendication 1 ou la revendication 2, dans lequel l'organisme hôte est une souche de E. coli.

5. Procédé suivant la revendication 1 ou la revendication 2, dans lequel l'organisme hôte est issu d'un mammifère.

6. Procédé suivant la revendication 5, dans lequel l'organisme hôte est une lignée de cellules d'ovaires de hamster chinois.

7. Procédé suivant l'une quelconque des revendications 1 à 6, qui comprend les étapes consistant à recueillir et purifier la protéine.

8. Procédé qui, après la préparation d'une protéine ayant la fonction de l'activateur du plasminogène tissulaire par un procédé suivant l'une quelconque des revendications 1 à 7, comprend l'utilisation de ladite protéine dans la production d'une préparation pharmaceutique.

9. Procédé qui comprend la préparation d'un isolat d'ADN codant pour une telle protéine préparée par le procédé suivant la revendication 1 ou la revendication 2.

10. Procédé qui comprend la préparation d'un vecteur de clonage recombinant contenant l'ADN codant pour une telle protéine préparée par le procédé suivant la revendication 1 ou la revendication 2.

11. Procédé qui comprend la préparation d'un vecteur d'expression recombinant qui contient l'ADN codant pour une telle protéine préparée par le procédé suivant la revendication 1 ou la revendication 2, et qui est capable, dans une culture de micro-organismes ou cellules transformants, d'exprimer la séquence codante pour produire le polypeptide correspondant.

12. Culture de micro-organismes ou de cellules transfectée avec l'ADN codant pour une telle protéine préparée par le procédé suivant la revendication 1 ou la revendication 2, et capable d'exprimer cet ADN pour produire ladite protéine.

13. Micro-organisme suivant la revendication 12, qui est une souche de E. coli.

14. Culture cellulaire suivant la revendication 12, qui est une lignée cellulaire de mammifère.

15. Culture cellulaire suivant la revendication 14, dans laquelle la lignée cellulaire est une lignée de cellules d'ovaires de hamster chinois.
